(19) Europäisches Patentamt
European Patent Office
Office européen des brevets

(11) Veröffentlichungsnummer: **0 008 732**
**A2**

# (12) EUROPÄISCHE PATENTANMELDUNG

(21) Anmeldenummer: **79103043.0**

(22) Anmeldetag: **20.08.79**

(51) Int. Cl.³: **A 01 N 43/30**
A 01 N 43/32, A 01 N 37/28
C 07 C 69/734, C 07 C 121/75
C 07 C 149/40, C 07 D 319/20
C 07 C 67/11, C 07 C 120/00
C 07 C 148/00, C 07 C 67/14
//C07C43/225, C07C41/22,
C07C149/34

(30) Priorität: **28.08.78 DE 2837524**
**14.10.78 DE 2844816**

(43) Veröffentlichungstag der Anmeldung:
**19.03.80 Patentblatt 80/6**

(84) Benannte Vertragsstaaten:
**AT BE CH DE FR GB IT NL SE**

(71) Anmelder: **BAYER Aktiengesellschaft**
**Zentralbereich Patente,Marken und Lizenzen Bayerwerk**
**D-5090 Leverkusen 1(DE)**

(72) Erfinder: **Fuchs, Rainer, Dr.**
**Roeberstrasse 8**
**D-5600 Wuppertal 1(DE)**

(72) Erfinder: **Klauke, Erich, Dr.**
**Eichendorffweg 8**
**D-5068 Odenthal(DE)**

(72) Erfinder: **Hammann, Ingeborg, Dr.**
**Belfortstrasse 9**
**D-5000 Köln(DE)**

(72) Erfinder: **Homeyer, Bernhard, Dr.**
**Obere Strasse 28**
**D-5090 Leverkusen 3(DE)**

(72) Erfinder: **Behrenz, Wolfgang, Dr.**
**Untergruendemich 14**
**D-5063 Overath(DE)**

(72) Erfinder: **Stendel, Wilhelm, Dr.**
**In dem Birken 55**
**D-5600 Wuppertal 1(DE)**

(72) Erfinder: **Lantzsch, Reinhard, Dr.**
**Heymannstrasse 32**
**D-5090 Leverkusen(DE)**

(72) Erfinder: **Marhold, Albrecht, Dr.**
**Carl-Duisberg-Strasse 329**
**D-5090 Leverkusen 1(DE)**

## (54) Fluorhaltige Phenylessigsäureester, Verfahren zu deren Herstellung, deren Verwendung als Insektizide und Akarizide; Zwischenprodukte zu deren Herstellung und Herstellung dieser Zwischenprodukte.

(57) Die Erfindung betrifft neue fluorhaltige Phenylessigsäureester der Formel I

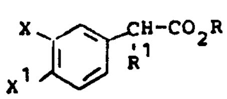

(I)

Sie werden erhalten, wenn man Säuren der Formel (II)

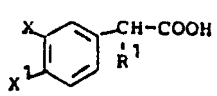

(II)

oder reaktionsfähige Derivate derselben, mit Alkoholen der Formel R - Z² oder mit einem reaktionsfähigen Derivat derselben, gegebenenfalls in Gegenwart eines Säureakzeptors und gegebenenfalls unter Verwendung eines Verdünnungsmittels umsetzt.

Die neuen fluorhaltigen Phenylessigsäureester der Formel (I) zeichnen sich durch hohe insektizide und akarizide Wirksamkeit aus.

EP 0 008 732 A2

BAYER AKTIENGESELLSCHAFT 5090 Leverkusen, Bayerwerk
Zentralbereich
Patente, Marken und Lizenzen Rt/kl/Kü
Typ 1a

**BEZEICHNUNG GEÄNDERT**
**siehe Titelseite**

Fluorhaltige Phenylessigsäureester, Verfahren zu deren Herstellung, deren Verwendung als Insektizide und Akarizide und Zwischenprodukte zu deren Herstellung

Die vorliegende Erfindung betrifft neue fluorhaltige Phenylessigsäureester, Verfahren zu ihrer Herstellung und deren Verwendung als Insektizide und Akarizide, sowie neue Zwischenprodukte zur Herstellung dieser Wirkstoffe.

Phenylessigsäureester sind bereits bekannt aus den deutschen Auslege- bzw. Offenlegungsschriften DE-AS 23 35 347 und DE-OS 27 43 416.

Die daraus bekannten Verbindungen besitzen jedoch entweder den Nachteil zu geringer Wirksamkeit, insbesondere was ihr Wirkungsspektrum betrifft oder aber sie sind im großtechnischen Maßstab wirtschaftlich nicht herzustellen.

1. Es wurden die neuen fluorhaltigen Phenylessigsäureester der Formel (I) gefunden

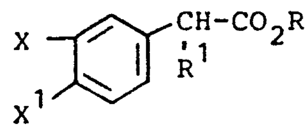

(I)

in welcher

R für den Rest eines bei Pyrethroiden üblichen Alkohols steht,

$R^1$ für $C_{2-4}$-Alkyl, $C_{2-4}$-Alkenyl oder Cyclopropyl steht,

X für H, Halogen, Alkyl, Alkoxy, $OCHF_2$, $SCHF_2$, $SCClF_2$, $SCF_3$ steht,

$X^1$ für den Fall, daß R für gegebenenfalls durch α-Cyano, oder α-Äthinylsubstituierten 4-Fluor-3-phenoxybenzylalkohol steht, für Halogenalkoxy oder Halogenalkylthio oder gemeinsam mit X für fluorsubstituiertes Methylendioxy oder Ethylendioxy steht und zusätzlich für den Fall, daß $R^1$ für Cyclopropyl steht und R für gegebenenfalls durch α-Cyano oder α-Äthinyl substituierten 4-Fluor-3-phenoxybenzylalkohol steht, für Wasserstoff, Halogen, Alkyl, Halogenalkyl, Alkoxy, Alkylthio oder gemeinsam mit x für gegebenenfalls F substituiertes Methylendioxy oder für gegebenenfalls F substituiertes Ethylendioxy steht und in den Fällen, in denen R für andere bei Pyrethroiden übliche Alkohole steht als für gegebenenfalls durch α-Cyano oder α-Äthinylsubstituierten 4-Fluor-3-phenoxybenzylalkohol, für Wasserstoff, $OCHF_2$, $SCHF_2$, $SCClF_2$, $SCF_3$, sowie ge-

gemeinsam mit X für $-OCH_2CF_2O-$ oder $-OCHFCF_2O-$ steht wobei in diesen Fällen X für $OCHF_2$, $SCHF_2$, $SCClF_2$ oder $SCF_3$ stehen muß wenn $X^1$ für Wasserstoff steht.

Die allgemeine Formel (I) schließt die verschiedenen möglichen Stereoisomeren und ihre Mischungen mit ein.

2. Es wurde ferner gefunden, daß man die neuen fluorhaltigen Phenylessigsäureester der Formel (I) erhält, indem man eine Säure oder deren reaktionsfähiges Derivat der Formel (II)

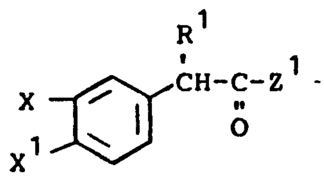

(II)

in welcher

X und $R^1$ die unter 1. (oben) angegebene Bedeutung haben und

$Z^1$ Halogen, vorzugsweise Fluor oder Chlor, OH oder $OC_{1-4}$-Alkyl bedeutet,

mit einem Alkohol oder dessen reaktionsfähigem Derivat der Formel (III)

$$R - Z^2 \quad \text{(III)}$$

in welcher

R die unter 1. (oben) angegebene Bedeutung hat und

$Z^2$ OH, Cl oder Br bedeutet,

gegebenenfalls in Gegenwart von Lösungsmitteln, Säureakzeptoren und/oder Phasentransferkatalysatoren oder in Gegenwart von Umesterungskatalysatoren umsetzt.

3. Es wurden ferner die neuen Säuren bzw. die reaktionsfähigen Derivate derselben der Formel (XII) gefunden,

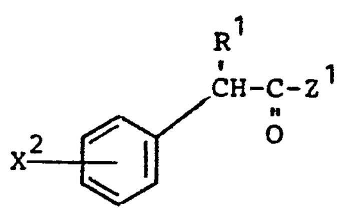

(XII)

in welcher

$X^2$ in 3 und/oder 4 Stellung für die Reste $-OCHF_2$, $-SCHF_2$, $-SCClF_2$, $-SCF_3$ sowie in 3 und 4 Stellung für die Reste $-OCH_2CF_2O-$ oder $-OCHFCF_2O-$, steht,

$R^1$ für $C_2$-$C_4$-Alkyl $C_{2-4}$-Alkenyl oder Cyclopropyl steht und

$Z^1$ die unter 2. (oben) angegebene Bedeutung besitzen.

4. Es wurde ferner gefunden, daß man die neuen Säuren, bzw. die reaktionsfähigen Derivate derselben, der Formel (XII) erhält, indem man die Verbindungen der Formel (IV)

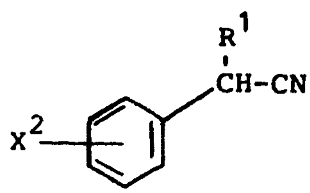

(IV)

in welcher

$X^2$ und $R^1$ die unter 3. (oben) angegebene Bedeutung besitzen,

mit einem Alkohol der Formel (V)

$$C_{1-4}\text{-Alkyl-OH}$$ (V)

in Gegenwart eines sauren Katalysators umsetzt und den entstandenen Iminoester zum Ester hydrolysiert, diesen gegebenenfalls zur Säure verseift und diese gegebenenfalls mit einem Halogenierungsmittel umsetzt oder für den Fall, daß $X^2$ in 3 und 4 Stellung für die Reste $-OCH_2CF_2O-$, $-OCHFCF_2O-$ steht, die CN-Gruppe der Verbindung der Formel (IV) zur COOH-Gruppe verseift und diese gegebenenfalls mit einem Halogenierungsmittel umsetzt.

5. Es wurden ferner die neuen Verbindungen der Formel (IV) gefunden

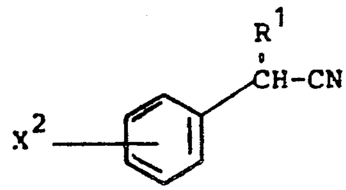

(IV)

in welcher

$X^2$ und $R^1$ die unter 3. (oben) angegebene Bedeutung besitzen.

6. Es wurde ferner gefunden, daß man die neuen Verbindungen der Formel (IV) erhält, indem man die Verbindungen der Formel (VI)

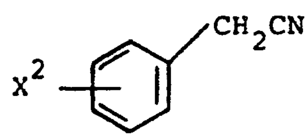

(VI)

in welcher

$X^2$ die unter 3. (oben) angegebene Bedeutung besitzt

mit einer Verbindung der allgemeinen Formel (VII)

$R^1$-Hal (VII)

in welcher

$R^1$ die unter 3. (oben) angegebene Bedeutung besitzt und

Hal für Cl, Br oder J steht,

in Gegenwart einer Base, sowie gegebenenfalls in Gegenwart eines Lösungsmittels und/oder eines Phasentransferkatalysators umsetzt.

7. Es wurden ferner die neuen Verbindungen der Formel (VI) gefunden

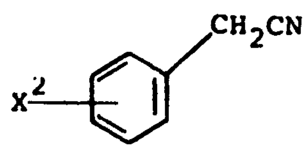

(VI)

in welcher

$X^2$ die unter 3. (oben) angegebene Bedeutung besitzt.

8. Es wurde ferner gefunden, daß man die neuen Verbindungen der Formel (VI) erhält, indem man die Verbindungen der Formel (VIII)

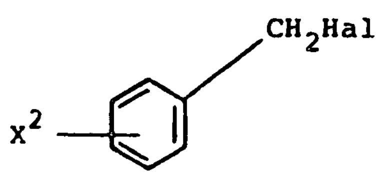

(VIII)

in welcher

X die unter 3. (oben) angegebene Bedeutung besitzt und

Hal für Cl oder Br steht,

mit Cyanwasserstoff oder vorzugsweise mit einem Alkalicyanid, gegebenenfalls in Gegenwart eines Katalysators umsetzt.

9. Es wurde ferner gefunden, daß man die Säurederivate der Formel (II)

in welcher

X und $X^1$ in 3 und 4 Stellung für den Rest $-OCF_2O-$ steht,

$Z^1$ für Fluor steht und

$R^1$ für $C_2$-$C_4$-Alkyl steht,

erhält, indem man eine Verbindung der Formel (IX)

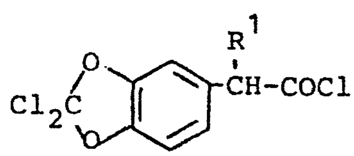

(IX)

in welcher

$R^1$ für $C_{2-4}$-Alkyl steht

mit wasserfreier Flußsäure umsetzt.

10. Es wurden ferner die neuen Verbindungen der Formel (IX) gemäß 9. (oben) gefunden.

11. Es wurde ferner gefunden, daß man die neuen Verbindungen der Formel (IX) gemäß 9. (oben) erhält, indem man eine Verbindung der Formel (X)

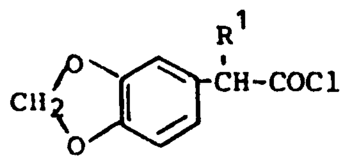

(X)

in welcher

$R^1$ für $C_{2-4}$-Alkyl steht

mit einem Chlorierungsmittel umsetzt.

Die Verbindungen der Formel (I) gemäß 1. (oben) zeigen gute insektizide und akarizide Eigenschaften.

Überraschenderweise zeigen die neuen erfindungsgemäßen Wirkstoffe gemäß 1. (oben) eine höhere sowie erheblich breitere Wirksamkeit als die aus dem Stand der Technik bekannten Verbindungen.

Bevorzugte Verbindungen der Formel (I) sind substituierte α-Phenyl-carbonsäure-(4-fluoro-3-phenoxy-benzyl)-ester der Formel (XI)

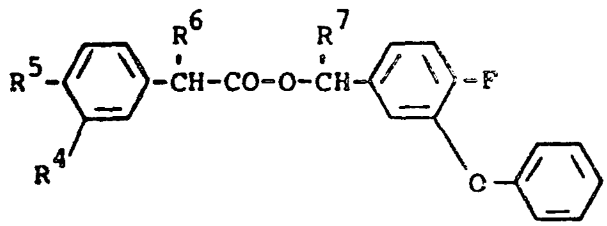

(XI)

in welcher

$R^4$ für Wasserstoff, Halogen, Alkyl oder Alkoxy steht,

$R^5$ für Halogenalkoxy oder Halogenalkylthio steht und - für den Fall, daß $R^6$ für Cyclopropyl steht - zusätzlich für Wasserstoff, Halogen, Alkyl, Halogenalkyl, Alkoxy, Alkylthio oder zusammen mit dem Rest $R^4$ für einen Methylendioxyrest steht,

$R^6$ für Äthyl, n-Propyl, iso-Propyl, iso-Propenyl oder Cyclopropyl steht und

$R^7$ für Wasserstoff, Cyano oder Äthinyl steht.

Besonders bevorzugt sind dabei α-Phenyl-carbonsäure-(4-fluoro-3-phenoxy-benzyl)-ester der Formel XI) in welcher

$R^4$ für Wasserstoff, Fluor, Chlor oder Brom sowie für Alkyl oder Alkoxy, letztere jeweils mit 1 oder 2 Kohlenstoffatomen, steht,

$R^5$ für Fluor- oder Chlorfluor-alkoxy sowie Fluor- oder Chlorfluor-alkylthio, jeweils mit 1 oder 2 Kohlenstoffatomen, steht und - für den Fall daß $R^6$ für Cyclopropyl steht - zusätzlich für Wasserstoff, Fluor, Chlor oder Brom sowie für Alkyl, Fluor- oder Chlorfluoralkyl, Alkoxy oder Alkylthio - letztere jeweils mit 1 oder 2 Kohlenstoffatomen - steht oder worin - ebenfalls für den Fall, daß $R^6$ für Cyclopropyl steht - $R^4$ und $R^5$ zusammen für Methylendioxy stehen,

$R^6$ für iso-Propyl oder Cyclopropyl steht und

$R^7$ für Wasserstoff, Cyano oder Äthinyl steht.

Ganz besonders bevorzugt sind dabei Verbindungen der Formel (XI), in welcher

$R^4$ für Wasserstoff, Chlor, Methyl oder Methoxy steht,

$R^5$ für Trifluormethoxy oder Trifluormethylthio steht und - für den Fall, daß $R^6$ für Cyclopropyl steht - zusätzlich für Wasserstoff, Fluor, Chlor, Brom, Methyl, Trifluormethyl, Methoxy oder Methylthio steht, oder worin - ebenfalls für den Fall, daß $R^6$ für Cyclopropyl steht - R und $R^1$ zusammen für Methylendioxy stehen,

$R^6$ für iso-Propyl oder Cyclopropyl steht und

$R^7$ für Wasserstoff, Cyano oder Äthinyl steht.

Weiter sind von den erfindungsgemäßen Verbindungen der Formel (I) gemäß 1. (oben) diejenigen bevorzugt, in denen $R^1$ für Äthyl oder Isopropyl steht X und $X^1$ die unter 1 (oben) angegebene Bedeutung besitzen und R für

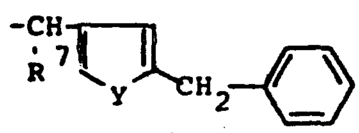

oder

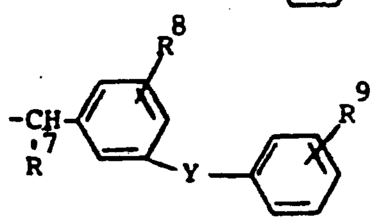

steht, wobei

$R^7$ H, CN oder -C≡CH steht,

Y für O oder S steht und

$R^8$ und $R^9$ für H oder Halogen, vorzugsweise für Fluor stehen.

Besonders bevorzugt sind Verbindungen in denen R für

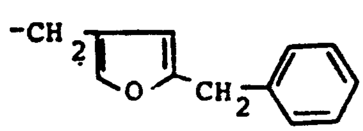

oder

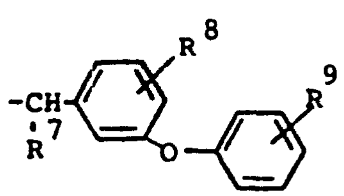

steht, wobei $R^7$, $R^8$ und $R^9$ die oben angegebene Bedeutung besitzen.

Folgende Verbindungen der Formel (I) seien im einzelnen genannt:

3'-Phenoxybenzyl-α-isopropyl-3-difluormethoxy-phenylacetat,
3'-Phenoxybenzyl-α-isopropyl-4-difluormethoxy-phenylacetat,
3'-Phenoxybenzyl-α-isopropyl-3-trifluormethylthio-phenylacetat,
3'-Phenoxybenzyl-α-isopropyl-4-trifluormethylthio-phenylacetat,
3'-Phenoxybenzyl-α-isopropyl-3-difluormethylthio-phenylacetat,
3'-Phenoxybenzyl-α-isopropyl-4-difluormethylthio-phenylacetat,
3'-Phenoxybenzyl-α-isopropyl-3-difluorchlormethylthio-phenylacetat,
3'-Phenoxybenzyl-α-isopropyl-4-difluorchlormethylthio-phenylacetat,
3'-Phenoxy-(α'-cyano)benzyl-α-isopropyl-3-difluormethoxy-phenylacetat,
4'-Phenoxy-(α'-cyano)benzyl-α-isopropyl-4-difluormethoxy-phenylacetat,
3'-Phenoxy-(α'-cyano)benzyl-α-isopropyl-3-trifluormethylthio-phenylacetat,
3'-Phenoxy-(α'-cyano)benzyl-α-isopropyl-4-trifluormethylthio-phenylacetat,
3'-Phenoxy-(α'-cyano)benzyl-α-isopropyl-3-difluormethylthio-phenylacetat,
3'-Phenoxy-(α'-cyano)benzyl-α-isopropyl-4-difluormethylthio-phenylacetat,
3'-Phenoxy-(α'-cyano)benzyl-α-isopropyl-3-difluorchlormethylthio-phenylacetat,
3'-Phenoxy-(α'-cyano)benzyl-α-isopropyl-4-difluorchlormethylthio-phenylacetat,

5'-Benzyl-3'-furylmethyl-α-isopropyl-4-difluormethoxy-phenylacetat,

5'-Benzyl-3'-furylmethyl-α-isopropyl-4-trifluormethylthio-phenylacetat,

3'-Phenoxy-(α'-cyano)benzyl-α-äthyl-4-difluormethoxy-phenylacetat,

3'-Phenoxy-(α'-cyano)benzyl-α-äthyl-4-trifluormethylthio-phenylacetat,

3'-Phenoxybenzyl-α-äthyl-4'-trifluormethylthio-phenylacetat,

3'-Phenoxybenzyl-α-isopropyl-3,4-difluoräthylen-phenylacetat,

3'-Phenoxy-(α'-cyano)benzyl-α-isopropyl-3,4-difluoräthylendioxy-phenylacetat,

5'-Benzyl-3'-furylmethyl-α-isopropyl-3,4-difluoräthylendioxy-phenylacetat

3'-Phenoxybenzyl-α-äthyl-3,4-difluoräthylendioxy-phenylacetat,

3'-Phenoxy-(α'-cyano)benzyl-α-äthyl-3,4-difluoräthylendioxy-phenylacetat,

5'-Benzyl-3'-furylmethyl-α-äthyl-3,4-difluoräthylendioxy-phenylacetat,
3'-Phenoxybenzyl-α-isopropyl-3,4-trifluoräthylendioxy-phenylacetat,
3'-Phenoxy-(α'-cyano)benzyl-α-isopropyl-3,4-trifluoräthylendioxy-phenylacetat,
5'-Benzyl-3'-furylmethyl-α-isopropyl-3,4-trifluoräthylendioxy-phenylacetat,
3'-Phenoxybenzyl-α-äthyl-3,4-trifluoräthylendioxy-phenylacetat,
3'-Phenoxy-(α'-cyano)benzyl-α-äthyl-3,4-trifluoräthylendioxy-phenylacetat,
5'-Benzyl-3'-furylmethyl-α-äthyl-3,4-trifluoräthylendioxy-phenylacetat,
4'-Fluor-3'-phenoxy-(α'-cyano)benzyl-α-isopropyl-3,4-difluormethylendioxy-phenylacetat,
4'-Fluor-3'-phenoxy-(α'-cyano)benzyl-α-isopropyl-3,4-difluoräthylendioxy-phenylacetat,
4'-Fluor-3'-phenoxy-(α'-cyano)benzyl-α-isopropyl-3,4-trifluoräthylendioxy-phenylacetat.

Die Herstellung der erfindungsgemäßen fluorhaltigen Phenylessigsäureester kann durch folgendes Reaktionsschema wiedergegeben werden:

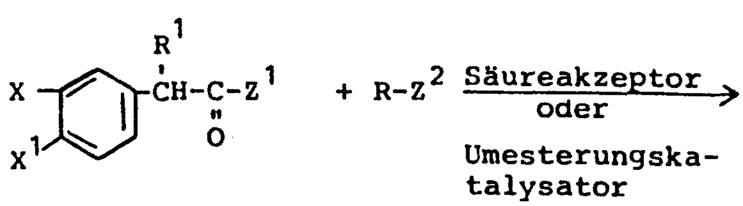

(I)

Verwendet man beispielsweise gemäß Verfahren 2. (oben)

4-Difluormethylthio-α-isopropyl-phenylessigsäurechlorid und 3-Phenoxy-α-cyano-benzylalkohol als Ausgangsmaterialien, so kann der Reaktionsverlauf durch das folgende Formelschema wiedergegeben werden:

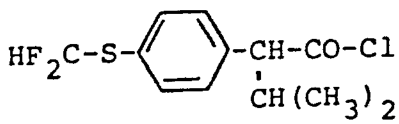

+

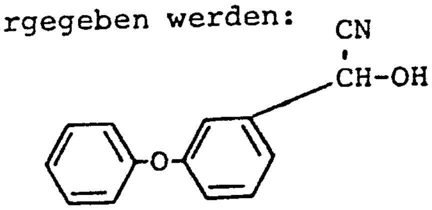

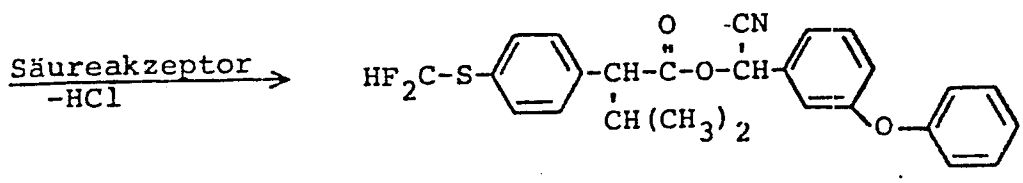


Verwendet man beispielsweise 3-Difluormethoxy-α-isopropyl-phenylessigsäure und 3-Phenoxy-benzylchlorid als Ausgangsstoffe, so kann der Reaktionsverlauf durch das folgende Formelschema wiedergegeben werden:

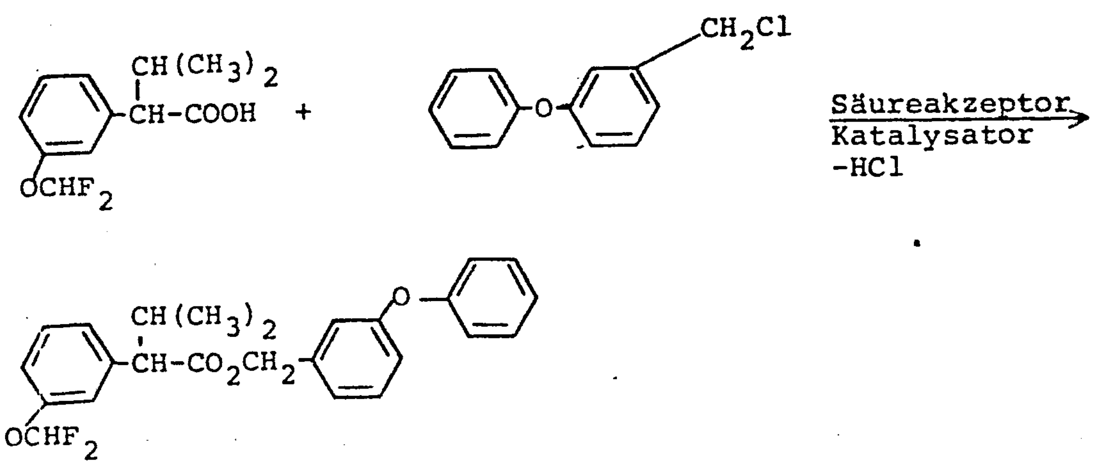

Verwendet man beispielsweise 4-Trifluormethylthio-α-isopropyl-phenylessigsäuremethylester und 4-Benzyl-3-furylmethanol als Ausgangsstoffe, so kann der Reaktionsverlauf durch das folgende Formelschema wiedergegeben werden:

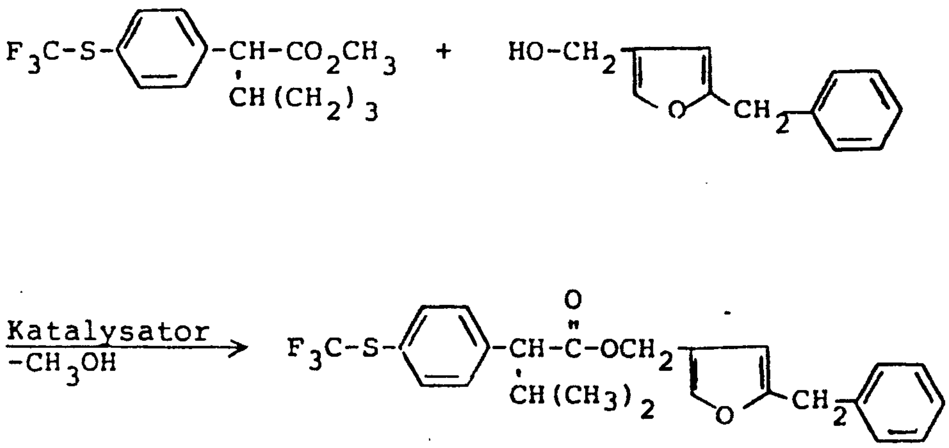


Die als Ausgangsprodukte zu verwendenden fluorhaltigen Phenylessigsäuren bzw. deren reaktionsfähige Derivate der Formel (II) sind zum Teil neu (vgl. 3. oben). Als reaktionsfähige Derivate werden bevorzugt die Säurechloride oder -fluoride eingesetzt. Ein anderer Teil dieser Säuren bzw. ihrer Derivate ist bekannt oder sie können analog zu bekannten Verfahren hergestellt werden (vgl. DE-OS 2 335 347, 2 717 414, 2 743 416).

α-Phenyl-α-cyclopropyl-essigsäure,
α-(4-Fluoro-phenyl)-α-cyclopropyl-essigsäure,
α-(4-Chloro-phenyl)-α-cyclopropyl-essigsäure,
α-(4-Bromo-phenyl)-α-cyclopropyl-essigsäure,
α-(4-Methyl-phenyl)-α-cyclopropyl-essigsäure,
α-(4-Trifluormethyl-phenyl)-α-cyclopropyl-essigsäure,

α-(4-Methoxy-phenyl)-α-cyclopropyl-essigsäure,
α-(4-Methylthio-phenyl)-α-cyclopropyl-essigsäure,
α-(4-Trifluormethoxy-phenyl)-α-cyclopropyl-essigsäure,
α-(4-Trifluormethylthio-phenyl)-α-cyclopropyl-essigsäure,
α-(3,4-Methylendioxy-phenyl)-α-cyclopropyl-essigsäure,
α-(3,4-Difluormethylendioxy-phenyl)-α-isopropyl-essigsäure
α-(4-Trifluormethylendioxy-phenyl)-α-isopropyl-essigsäure
und die entsprechenden Säurechloride.

Als Beispiele für die als Ausgangsprodukte zu verwenden neuen Verbindungen der Formel (XII) vergleiche 3. (oben) seien im einzelnen genannt:

3-Difluormethoxy-α-äthyl-phenylessigsäure (und -chlorid, -methylester, -äthylester),
4-Difluormethoxy-α-äthyl-phenylessigsäure (und -chlorid, -methylester, -äthylester),
3-Difluormethoxy-α-isopropyl-phenylessigsäure (und -chlorid, -methylester, -äthylester),
4-Difluormethoxy-α-isopropyl-phenylessigsäure (und -chlorid, -methylester, -äthylester),
3-Difluormethylthio-α-isopropyl-phenylessigsäure (und -chlorid, -methylester, -äthylester),
4-Difluormethylthio-α-isopropyl-phenylessigsäure (und -chlorid, -methylester, -äthylester),
4-Difluormethylthio-α-äthyl-phenylessigsäure (und -chlorid, -methylester, -äthylester),

4-Difluorchlormethylthio-α-äthyl-phenylessigsäure (und -chlorid, -methylester, -äthylester),
4-Difluorchlormethylthio-α-isopropyl-phenylessigsäure (und -chlorid, -methylester, -äthylester),
3-Trifluormethylthio-α-äthyl-phenylessigsäure (und -chlorid, -methylester, -äthylester),
3-Trifluormethylthio-α-isopropyl-phenylessigsäure (und -chlorid, -methylester, -äthylester),
4-Trifluormethylthio-α-isopropyl-phenylessigsäure (und -chlorid, -methylester, -äthylester),
4-Trifluormethylthio-α-äthyl-phenylessigsäure (und -chlorid, -methylester, -äthylester),
3,4-Difluoräthylendioxy-α-isopropyl-phenylessigsäure (und -chlorid, -methylester, -äthylester),
3,4-Trifluoräthylendioxy-α-äthylphenylessigsäure (und -chlorid, -methylester, -äthylester),
3,4-Trifluoräthylendioxy-α-isopropyl-phenylessigsäure (und -chlorid, -methylester, -äthylester),
3,4-Difluoräthylendioxy-α-äthyl-phenylessigsäure( und -chlorid, -methylester, -äthylester).

Die neuen Verbindungen der Formel (XII) können nach dem unter 4. (oben) angegebenen Verfahren hergestellt werden (Einzelheiten siehe weiter unten).

Die ebenfalls als Ausgangsprodukte zu verwendenden Alkohole bzw. reaktionsfähigen Derivate der Formel (III) sind bekannt und nach allgemein üblichen, in der Literatur beschriebenen Verfahren herstellbar (vgl. z.B. DE-AS oder DE-OS 25 54 883, 19 26 433, 26 12 115, 24 36 178, 24 36 462, sowie Monatshefte 67, Seite 35, [1936]).

4-Fluor-3-phenyl-benzylalkohol bzw. sein α-Cyano oder α-Äthinylderivat erhält man ausgehend vom literaturbekannten 3-Bromo-4-fluoro-toluol beispielsweise gemäß folgendem Reaktionsschema:

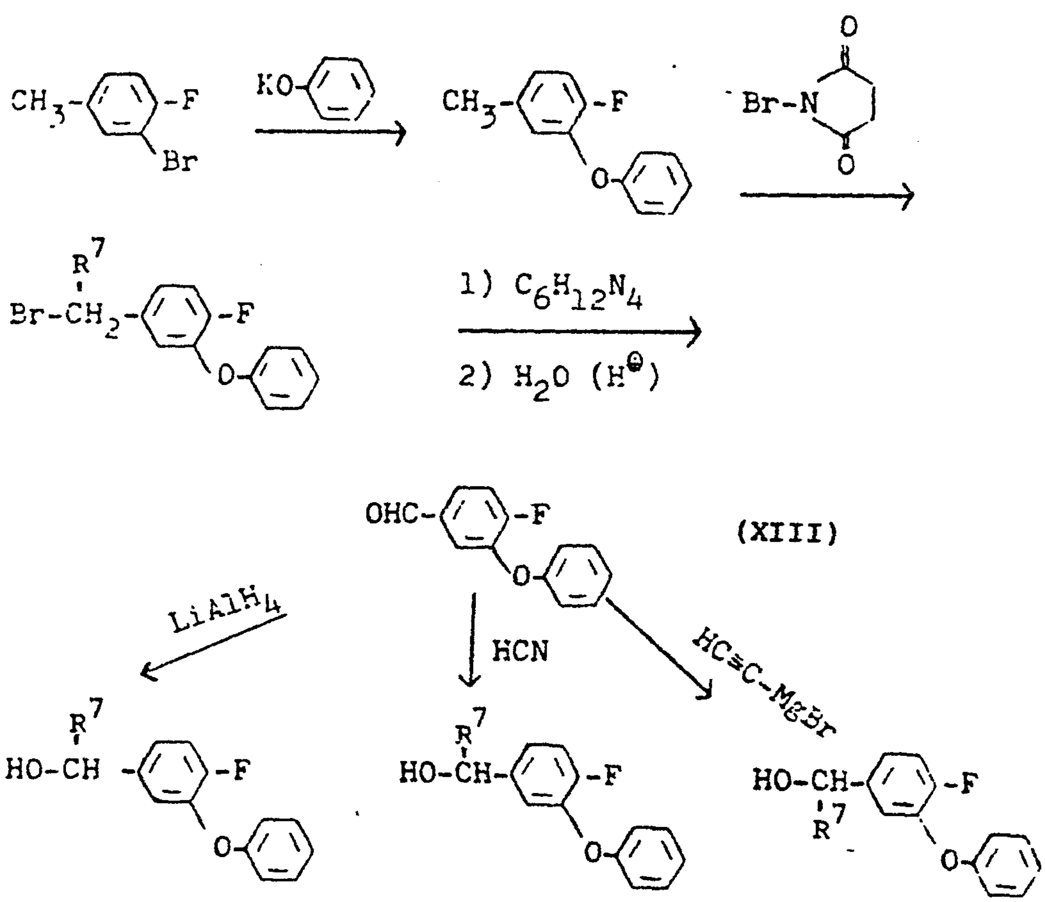


XIV a
$R^7$ = H

XIV b
$R^7$ = CN

XIV c
$R^7$ = $C\equiv CH$

4-Fluoro-3-phenoxy-toluol wird aus 3-Bromo-4-fluoro-toluol vorteilhaft durch Umsetzung mit überschüssigem Kaliumphenolat in Gegenwart einer katalytischen Menge Kupferoxid und unter Verwendung von Dimethylformamid als Verdünnungsmittel bei Reaktionstemperaturen zwischen 12o und 1δo°C hergestellt. Die Reaktion des so erhaltenen Zwischenproduktes mit N-Brom-succinimid in Gegenwart eines Radikalstarters wie z.B. Azodiisobuttersäurenitril, gegebenenfalls unter Verwendung eines Verdünnungsmittels, wie z.B. Tetrachlorkohlenstoff, bei Temperaturen zwischen 5o und 1oo°C führt zu 4-Fluoro-3-phenoxy-benzylbromid. Daraus kann in einer Sommelet-Reaktion, d.h. durch Umsetzung mit Hexamethylentetramin in Gegenwart eines Verdünnungsmittels wie z.B. Methylenchlorid bei Temperaturen zwischen 0 und 5o°C, anschließend mit wässriger Essigsäure und im Anschluß daran mit Salzsäure bei Temperaturen zwischen 8o und 12o°C 4-Fluoro-3-phenoxy-benzaldehyd (XII) hergestellt werden.

Aus dem Aldehyd (XIII) erhält man die entsprechenden Alkohole der Formel (XIV) die von der Formel (III) (oben) umfaßt werden, je nach der Bedeutung von $R^7$ nach verschiedenen Methoden:

a) für den Fall, daß $R^7$ für Wasserstoff steht, durch Reaktion mit einem komplexen Metallhydrid wie z.B. Lithiumaluminiumhydrid in einem inerten Verdünnungsmittel wie z.B. Diäthyläther bei Temperaturen zwischen 0 und 50°C.

b) für den Fall, daß $R^7$ für Cyano steht, durch Umsetzung mit einem Alkalicyanid wie z.B. Natriumcyanid in Gegenwart einer Säure wie z.B. Essigsäure, welche gegebenenfalls mit Wasser verdünnt ist bei Temperaturen zwischen 0 und 5o°C und

c) für den Fall, daß $R^7$ für Äthinyl steht, durch Umsetzung mit einer Äthinylverbindung wie z.B. Äthinylmagnesiumbromid, gegebenenfalls unter Verwendung eines inerten Verdünnungsmittels wie z.B. Diäthyläther bei Temperaturen zwischen 0 und 5o°C.

Bevorzugt sind Verbindungen der Formel (III) in welcher R die weiter oben angegebene bevorzugte Bedeutung hat.

Als Beispiele für die als Ausgangsprodukte zu verwendenden Verbindungen der Formel (III) seien im einzelnen genannt:

5-Benzyl-3-hydroxymethyl-furan,
5-Benzyl-2-hydroxymethyl-furan,
5-Benzyl-3-hydroxymethyl-thiophen,
5-Phenoxy-5-hydroxymethyl-furan,
3-Hydroxy-4-methyl-5-allyl-cyclopenten(4)-1-on,
N-Hydroxymethyl-phthalimid,
N-Hydroxymethyl-3,4,5,6-tetrahydrophthalimid,
Pentafluorbenzylalkohol,

4-Phenyl-3-chlor-2-buten-1-ol,
3-Trifluormethoxybenzylalkohol,
3-Dichlorvinyloxybenzylalkohol,
3-Propargyloxybenzylalkohol,
3-Dichlorvinyloxy-$\alpha$-cyano-benzylalkohol,
3-Phenoxy-benzylalkohol,
3-Phenyl-$\alpha$-cyano-benzylalkohol,
3-Phenoxy-$\alpha$-methoxycarbonyl-benzylalkohol,
3-Phenoxy-$\alpha$-äthinyl-benzylalkohol,
3-Phenoxy-4-fluor-benzylalkohol,
3-Phenoxy-4-chlor-benzylalkohol,
3-Phenoxy-4-fluor-$\alpha$-cyanobenzylalkohol,
3-(4'-Fluorphenoxy)-benzylalkohol,
3-(4'-Chlorphenoxy)-benzylalkohol,
3-(4'-Bromphenoxy)-benzylalkohol,
3-Difluormethoxy-benzylchlorid,
3-Phenoxy-benzylbromid,
Pentafluorbenzylchlorid.

Zur Herstellung der erfindungsgemäßen Verbindungen der Formel (I) gemäß 1. (oben) aus Carbonsäuren bzw. Carbonsäurehalogeniden der Formel (II) und Alkoholen bzw. Chloriden oder Bromiden der Formel (III) können als Säureakzeptoren alle üblichen Säurebindemittel Verwendung finden.

Besonders bewährt haben sich Alkalihydroxide, -carbonate und -alkoholate, wie Kaliumhydroxid, Natriumhydroxid, Natriummethylat, Kaliumcarbonat, Natriumäthylat, ferner aliphatische, aromatische oder heterocyclische Amine, beispielsweise Triäthylamin, Trimethylamin,

Dimethylanilin, Dimethylbenzylamin und Pyridin.

Die Reaktionstemperatur kann innerhalb eines größeren Bereiches variiert werden. Im allgemeinen arbeitet man bei der Umsetzung der Säurehalogenide mit Alkoholen zwischen 0 und 100°C, vorzugsweise bei 15 bis 40°C und bei der Umsetzung der Carbonsäure mit den Halogeniden zwischen 50 und 150°C, vorzugsweise bei 80°C bis 120°C. Im letzteren Falle wird bevorzugt in Gegenwart eines Katalysators gearbeitet.

Als Katalysator kommen alle sogenannten Phasentransferkatalysatoren in Betracht, wie beispielsweise Kronenäther oder quartäre Ammonium- oder Phosphoniumsalze. Bevorzugt sind quartäre Ammoniumsalze, wie beispielsweise Tetrabutylammoniumchlorid,Tetrabutylammoniumbromid, Benzyltriäthylammoniumchlorid oder Methyltrioctylammoniumchlorid.

Die Umsetzung läßt man im allgemeinen bei Normaldruck ablaufen. Das Verfahren zur Herstellung der erfindungsgemäßen Verbindungen wird bevorzugt unter Mitverwendung geeigneter Lösungs- und Verdünnungsmittel durchgeführt. Als solche kommen praktisch alle inerten organischen Solventien in Frage. Hierzu gehören insbesondere aliphatische und aromatische, gegebenenfalls chlorierte Kohlenwasserstoffe wie Benzol, Toluol, Xylol, Benzin, Methylenchlorid, Chloroform, Dichloräthan, Chlorbenzol, o-Dichlorbenzol oder Äther, z.B. Diäthyl-, Diisopropyl oder Dibutyläther, außerdem Nitrile, wie Aceto- und Propionitril.

Zur Durchführung des Verfahrens setzt man die Ausgangskomponenten vorzugsweise in äquimolaren Verhältnissen ein. Die Reaktionskomponenten werden im allgemeinen in einem der angegebenen Lösungsmittel zusammengegeben und meist bei erhöhter Temperatur nach Zugabe des Säureakzeptors und gegebenenfalls des Katalysators zur Vervollständigung der Reaktion eine oder mehrere Stunden gerührt. Anschließend gießt man das Reaktionsgemisch in Wasser, trennt die organische Phase ab und wäscht diese mit Wasser neutral. Nach dem Trocknen wird das Lösungsmittel im Vakuum abdestilliert. Die neuen Verbindungen fallen in Form von Ölen an, die sich zum Teil nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Die erfindungsgemäßen Verbindungen der Formel (I) lassen sich auch durch Umesterung aus den Estern der Formel (II) ($Z^1$ $=OC_{1-4}$-Alkyl) sowie den Alkoholen der Formel (III) gewinnen. In diesem Falle ist kein Säureakzeptor notwendig; der niedriger siedende Alkohol wird laufend aus dem Reaktionsansatz herausdestilliert. Die Reaktion wird bevorzugt in Gegenwart von Katalysatoren durchgeführt. Als Umesterungskatalysatoren kommen sowohl saure als auch basische Katalysatoren in Frage. Bevorzugt sind basische Katalysatoren wie z.B. Alkoholate. Als Beispiele seien genannt: $NaOCH_3$; $NaOC_2H_5$, Kalium-tert.-butylat, Titantetrabutylat.

Die neuen Säuren, Säurehalogenide bzw. Ester der Formel (XII) lassen sich nach dem unter 4. (oben) angegebenen Verfahren aus den Verbindungen IV herstellen.

Für den Fall, daß $X^2$ in und/oder 4 Stellung für die Reste $-OCHF_2$, - $SCHF_2$, $-SCClF_2$, $-SCF_3$ steht, würden die Reste $X^2$ drastische Bedingungen, wie sie zur Verseifung (Hydrolyse) eines Nitrils zur Carbonsäure notwendig sind, nicht unverändert überstehen, daher können die Verbindungen IV in diesen Fällen nicht direkt durch Hydrolyse in die Verbindungen XII mit $Z^1$=OH umgewandelt werden.

Aufgabe des erfindungsgemäßen Verfahrens war es daher, für diese Fälle eine Methode zu finden, die es gestattet, bei möglichst milden Bedingungen, unter denen diese Reste $X^2$ unverändert bleiben, eine Umwandlung der Nitrile zu erreichen.

Es wurde nun gefunden, daß sich die Nitrilgruppe in eine Estergruppe umwandeln läßt, ohne diese Reste $X^2$ zu zerstören, indem man die Nitrile der Formel (IV) in einem Alkohol der Formel (V) $C_{1-4}$-Alkyl-OH löst und die Lösung bei etwa -10 - 50°C, vorzugsweise bei 0 - 20°C, mit Chlorwasserstoff sättigt. Nach einigem Stehen bildet sich der Iminoester, aus dem mit Wasser der Ester entsteht. Es kann auch gleich in wäßriger Lösung gearbeitet werden.

Der Ester kann dann direkt durch Umesterung in eine Verbindung der Formel (I) umgewandelt werden oder in üblicher Weise zur Säure verseift werden und gegebenenfalls mit den üblichen Halogenierungsmitteln, wie beispielsweise $SOCl_2$, $COCl_2$, $PCl_3$, $PCl_5$, Oxalylchlorid oder -bromid, $PBr_3$ zu den Säurehalogeniden umgesetzt werden.

Die Nitrilgruppe der Verbindungen der Formel (IV) in welcher $X^2$ in 3 und 4 Stellung für die Reste $-O-CH_2CF_2O-$, $-OCHFCF_2O-$ steht, läßt sich sauer oder alkalisch verseifen.

Für die Verseifung kommen alle üblichen Verseifungsmittel in Frage, wie beispielsweise Schwefelsäure, Essigsäure oder vorzugsweise Alkalilaugen. Die Verseifung wird bei erhöhter Temperatur durchgeführt, beispielsweise bei 50 - 160°C, vorzugsweise bei 80 - 135°C.

Es kann aber auch wie weiter oben beschrieben, die Nitrilgruppe in eine Estergruppe umgewandelt werden, indem man die Nitrile in einem Alkohol der Formel (V) $C_{1-4}$-Alkyl-OH löst und die Lösung bei etwa 0 - 20°C mit Chlorwasserstoff sättigt. Nach einigem Stehen bildet sich der Iminoester, aus dem mit Wasser der Ester entsteht. Es kann auch gleich in wäßriger Lösung gearbeitet werden.

Der Ester kann dann direkt durch Umesterung in eine Verbindung der Formel (I) umgewandelt werden oder in üblicher Weise zur Säure verseift werden und gegebenenfalls mit den üblichen Halogenierungsmitteln, wie beispielsweise $SOCl_2$; $COCl_2$, $PCl_3$, $PCl_5$, Oxalylchlorid oder -bromid, $PBr_3$ zu den Säureahlogeniden umgesetzt werden.

Als Beispiele für die neuen Verbindungen der Formel (IV) seien im einzelnen genannt:

3,4-Difluoräthylendioxy-α-äthyl-phenylacetonitril,
3,4-Difluoräthylendioxy-α-isopropyl-phenylacetonitril,
3,4-Trifluoräthylendioxy-α-äthyl-phenylacetonitril,
3,4-Trifluoräthylendioxy-α-isopropyl-phenylacetonitril,
3-Difluormethoxy-α-äthyl-phenylacetonitril,
4-Difluormethoxy-α-äthyl-phenylacetonitril,
3-Difluormethoxy-α-isopropyl-phenylacetonitril,
4-Difluormethoxy-α-isopropyl-phenylacetonitril,
4-Difluormethylthio-α-isopropyl-phenylacetonitril,
3-Difluormethylthio-α-isopropyl-phenylacetonitril,
3-Difluorchlormethylthio-α-isopropyl-phenylacetonitril,
4-Difluorchlormethylthio-α-isopropyl-phenylacetonitril,
3-Trifluormethylthio-α-äthyl-phenylacetonitril,
4-Trifluormethylthio-α-äthyl-phenylacetonitril,
3-Trifluormethylthio-α-isopropyl-phenylacetonitril,
4-Trifluormethylthio-α-isopropyl-phenylacetonitril.

Die neuen Verbindungen der Formel (IV) lassen sich nach dem unter 6. (oben) angegebenen Verfahren aus den Verbindungen der Formel (VI) durch Alkylierung mit den Verbindungen der Formel (VII) erhalten.

Diese Reaktion läßt sich durch folgendes Formelschema darstellen:

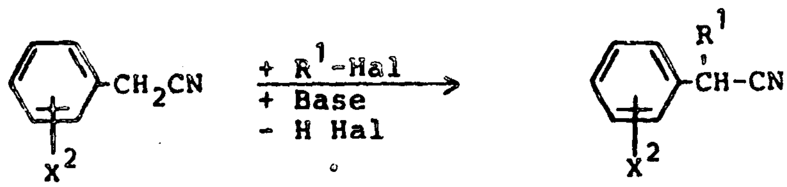


Die Alkylierung erfolgt in Gegenwart äquimolarer Mengen Base (bezogen auf $R^1$-Hal, das bevorzugt im Überschuß eingesetzt wird). Es kann mit oder ohne Lösungsmittel gearbeitet werden.

Als Basen kommen beispielsweise in Frage:

NaOH, KOH, $K_2CO_3$, Alkoholate, wie $NaOCH_3$, $NaOC_2H_5$, Natriumisopropylat oder Kalium-tert.-butylat.

Als Lösungsmittel kommen in erster Linie Alkohole in Frage wie beispielsweise Methanol, Äthanol, iso-Propanol. Bevorzugt wird - bei Verwendung von NaOH, KOH oder $K_2CO_3$ in Gegenwart von Phasentransferkatalysatoren gearbeitet. Als Phasentansferkatalysatoren kommen vor allem quartäre Ammoniumsalze, wie beispielsweise Tetrabutylammoniumchlorid und -bromid, Benzyltriäthylammoniumchlorid oder Methyltrioctylammoniumchlorid in Frage. Es kann in Gegenwart von Wasser, aber auch wasserfrei gearbeitet werden.

Als Beispiele für Verbindungen der Formel (VI) seien genannt:

3-Difluormethoxy-phenylacetonitril,
4-Difluormethoxy-phenylacetonitril,
3-Difluormethylthio-phenylacetonitril,
4-Difluormethylthio-phenylacetonitril,
3-Trifluormethylthio-phenylacetonitril,

4-Trifluormethylthio-phenylacetonitril,
3-Difluorchlormethylthio-phenylacetonitril,
4-Difluorchlormethylthio-phenylacetonitril,
3,4-Difluoräthylendioxy-phenylacetonitril,
3,4-Trifluoräthylendioxy-phenylacetonitril.

Als Beispiele für Verbindungen der Formel (VII) seien genannt:

Äthylenchlorid, Äthylbromid, Äthyljodid, 2-Chlorpropan, 2-Brompropan, 2-Jodpropan, 1-Brom-2-methyl-propan.

Die neuen Verbindungen der Formel (VI) werden nach dem Verfahren 8. (oben) aus den Verbindungen der Formel (VIII) mit Cyanwasserstoff oder vorzugsweise einem Alkalicyanid erhalten.

Die Verbindungen der Formel (VIII) sind bekannt oder können nach bekannten Verfahren erhalten werden. Verbindungen der Formel (VIII) in welcher $X^2$ in 3 und 4 Stellung für die Reste $-OCF_2O-$, $-OCH_2CF_2O-$, $-OCHFCF_2O-$ steht sind Gegenstand eines älteren nicht vorveröffentlichten Schutzbegehrens der Anmelderin (Deutsche Patentanmeldung P 28 19 788.8, Le A 18 825). Sie werden erhalten für den Fall, daß $X^2$ für $-OCF_2O-$ steht indem man die Verbindung der Formel

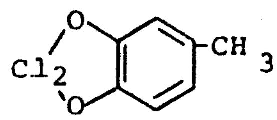

mit wasserfreier Flußsäure umsetzt oder für den Fall, daß X für $-OCH_2CF_2O-$, $-OCHFCF_2O-$ steht, indem man eine Verbindung der Formel

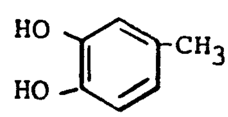

mit Verbindungen der Formel

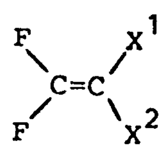

in welcher

$X^1$ für Halogen steht und

$X^2$ für Wasserstoff oder Halogen steht,

umsetzt und anschließend zum Benzylhalogenid halogeniert.

Das Verfahren zur Herstellung der Verbindungen der Formel (VI) wird durch folgendes Formelschema wiedergegeben:

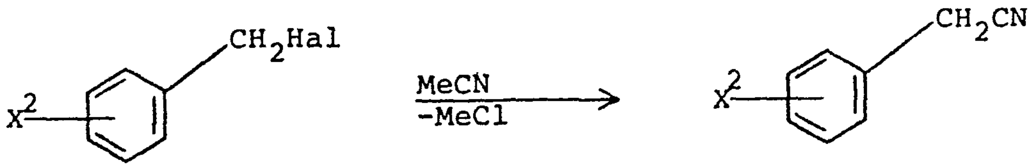


Me = Na, K

Als Halogenide werden die Chloride oder Bromide verwendet, als Cyanid wird vorzugsweise ein Alkalicyanid, wie Natrium- oder Kaliumcyanid verwendet.

Die Durchführung der Reaktion erfolgt nach im Prinzip bekannten Methoden (Houben-Weyl, Bd. VIII, Seite 294).

Mit Vorteil kann gegebenenfalls auch ohne Lösungsmittel mit Phasentransferkatalysatoren, wie z.B. quartären Ammoniumsalzen oder aber Aminen, wie Triäthylamin, Tripropylamin, Tributylamin gearbeitet werden (Synthesis 1973, Seite 448; Synthetic Comm. 6, 193 [1976]).

Säurederivate der Formel (XII) in welcher $X^2$ in 3 und 4 Stellung für den Rest $-OCF_2O-$ steht und $Z^1$ für Fluor steht und $R^1$ für $C_{2-4}$-Alkyl steht werden auch erhalten, indem man Verbindungen der Formel (IX) mit wasserfreier Flußsäure nach folgendem Formelschema umsetzt:

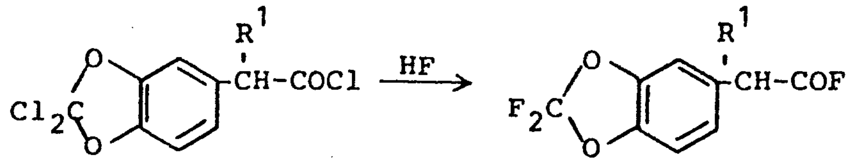

Die Umsetzung kann bei Temperaturen von -20°C bis 80°C erfolgen, besonders bevorzugt sind Temperaturen von 0°C bis 40°C.

Die Flußsäure muß mindestens in stöchiometrischen Mengen eingesetzt werden, doch ist im allgemeinen ein Überschuß günstig. So beträgt die Flußsäuremenge bevorzugt die zwei- bis dreifache stöchiometrische Menge, doch kann der Überschuß größer sein. Die Reaktion kann in Gegenwart von Lösungsmitteln wie auch ohne solche ausgeführt werden. Als Lösungsmittel kommen ganz allgemein inerte, aprotische Flüssigkeiten in Frage. Beispielsweise lassen sich Methylenchlorid, Trichlorfluormethan, Tetrachlorkohlenstoff, Chlorbenzol oder Nitrobenzol erfolgreich verwenden. Die Menge des Lösungsmittels ist für das erfindungsgemäße Verfahren nicht von Bedeutung. So wird die Reaktion vorzugsweise ohne Lösungsmittel durchgeführt, wenn die Ausgangsmaterialien flüssig sind.

Im allgemeinen erfolgt die Umsetzung bei Normaldruck, doch läßt sie sich auch bei Überdruck ausführen.

Das Verfahren kann wie folgt durchgeführt werden:

Im Reaktionsgefäß wird bei ca. -10°C die benötigte Menge an wasserfreier Flußsäure vorgelegt und unter

Rühren die Verbindungen der Formel (IX) zudosiert. Man wählt die Temperatur dann so, daß die Chlorwasserstoff-Entwicklung sofort einsetzt und leitet das entstehende Gas über einen Rückflußkühler in eine Vorlage, wo es kondensiert oder neutralisiert wird. Nach Ende der Zugabe kann die Temperatur noch etwas erhöht werden. Es wird bis zum Ende der Gasentwicklung gerührt und anschließend die überschüssige Flußsäure unter Normaldruck oder vermindertem Druck abdestilliert. Als Rückstand verbleigt das Reaktionsprodukt das dann beispielsweise durch Destillation gereinigt werden kann.

Die neuen Verbindungen der Formel (IX) werden nach dem Verfahren 10. (oben) durch Chlorierung der Verbindungen der Formel (X) erhalten. Die Verbindungen der Formel (X) sind bekannt (DE-OS 23 35 347).

Das Verfahren wird durch folgendes Reaktionsschema wiedergegeben:

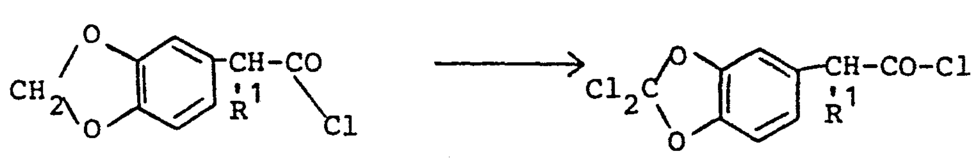

Als Chlorierungsmittel kommen Chlor und Phosphorpentachlorid in Frage. Es kann mit oder ohne Verdünnungsmittel gearbeitet werden. Als Verdünnungsmittel kommen beispielsweise in Frage: $PCl_3$; $POCl_3$; chlorhaltige Lösungsmittel, wie beispielsweise Tetrachlorkohlenstoff,

Chlorbenzol oder Dichlorbenzol. Bei Verwendung von Chlor wird bevorzugt unter radikalischen Bedingungen, z.B. unter Belichtung, gearbeitet.

Der Temperaturbereich liegt zwischen 50 und 150°C, bevorzugt zwischen 75 und 130°C.

Die Verbindungen der Formel (IX) können ohne weitere Reinigung in das unter 9. (oben) beschriebene Verfahren eingesetzt werden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen.Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta

migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra

Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von

brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Aryl-

sulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten auf dem veterinärmedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuders sowie durch parenterale Anwendung in Form beispielsweise der Injektion und im sogenannten "feed-through"-Verfahren.

Beispiel A

Laphygma-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der Wirkstoffzubereitung besprüht man Baumwollblätter (Gossypium hirsutum) taufeucht und besetzt sie mit Raupen des Eulenfalters (Laphygma frugiperda)

Nach den angegebenen Zeiten wird die Abtötung in % bestimmt. Dabei bedeutet 100 %, daß alle Raupen abgetötet wurden; 0 % bedeutet, daß keine Raupen abgetötet wurden.

Wirkstoffe, Wirkstoffkonzentrationen, Auswertungszeiten und Resultate gehen aus der nachfolgenden Tabelle hervor: 1, 9, 11.

Beispiel B

Tetranychus-Test (resistent)

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 3, 11, 16, 17, 18, 19, 23, 24.

Beispiel C

$LT_{100}$-Test für Dipteren

Testtiere: Aedes aegypti
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z.B. die folgenden Verbindungen der Herstellungsbeispiele überlegene Wirkung gegenüber dem Stand der Technik: 1, 2, 8, 9, 11, 16.

Beispiel D

Phaedon-Larven-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 16, 17, 18, 19, 23, 24.

Beispiel E

Grenzkonzentrations-Test / Bodeninsekten

Testinsekt: Phorbia antiqua-Maden (im Boden)
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm ( = mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 16, 17, 18, 19, 23, 24.

Beispiel F

Grenzkonzentrations-Test / Bodeninsekten

Testinsekt: Tenebrio molitor Larven (im Boden)
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm ( = mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 16, 23.

Beispiel G

$LD_{100}$-Test

Testtiere: Blatta orientalis
Zahl der Testtiere: 10
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100 %, daß alle Testtiere abgetötet wurden; 0 % bedeutet, daß keine Testtiere abgetötet wurden.

Bei diesem Test zeigen z.B. die folgenden Verbindungen der Herstellungsbeispiele überlegene Wirkung gegenüber dem Stand der Technik: 16.

Beispiel H

$LT_{100}$-Test für Dipteren

Testtiere: Musca domestica
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z.B. die folgenden Verbindungen der Herstellungsbeispiele überlegene Wirkung gegenüber dem Stand der Technik: 17.

Beispiel I

Test mit parasitierenden adulten Rinderzecken (Boophilus microplus res.)

Lösungsmittel: Alkylarylpolyglykoläther

Zur Herstellung einer zweckmäßigen Wirkstoffzubereitung vermischt man die betreffende aktive Substanz mit dem angegebenen Lösungsmittel im Verhältnis 1:2 und verdünnt das so erhaltene Konzentrat mit Wasser auf die gewünschte Konzentration.

10 adulte Rinderzecken (b.microplus res.) werden 1 Minute in die zu testende Wirkstoffzubereitung getaucht. Nach Überführung in Plastikbecher und Aufbewahrung in einem klimatisierten Raum wird der Abtötungsgrad bestimmt.

Bei diesem Test zeigen z.B. die folgenden Verbindungen der Herstellungsbeispiele überlegene Wirkung gegenüber dem Stand der Technik: 16, 17, 18, 19, 23.

Beispiel K

Test mit parasitierenden Fliegenlarven (Lucilia cuprina res.)

Emulgator: 80 Gewichtsteile Alkylarylpolyglykoläther

Zur Herstellung einer zweckmäßigen Wirkstoffzubereitung vermischt man 20 Gew.-Teile der betreffenden aktiven Substanz mit der angegebenen Menge des Emulgators und verdünnt das so erhaltene Gemisch mit Wasser auf die gewünschte Konzentration. Etwa 20 Fliegenlarven (Lucilia cuprina) werden in ein mit Wattestopfen entsprechender Größe beschicktes Teströhrchen gebracht, welches ca. 3 ml einer 20 %igen Eigelbpulver-Suspension in Wasser enthält. Auf diese Eigelbpulver-Suspension werden 0,5 ml der Wirkstoffzubereitung gebracht. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: 17, 19.

Herstellungsbeispiele:

1. Wirkstoffe der Formel (I)

Beispiel 1

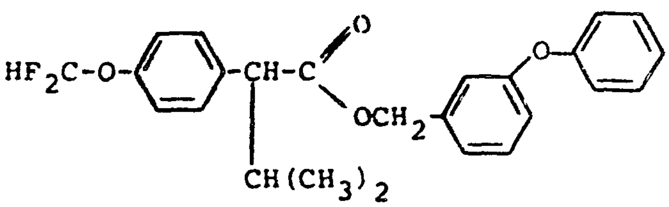

13 g 4-Difluormethoxy-α-isopropyl-phenylessigsäure, 8,7 g 3-Phenoxybenzylchlorid, 250 ml Toluol, 5 g Tetrabutylammoniumbromid und 3,2 g pulverisierte KOH (technisch, 88 %ig) werden gemischt und 4 h zum Sieden erhitzt. Nach dem Abkühlen wird die organische Phase abgetrennt und neutral gewaschen. Nach dem Trocknen mit Natriumsulfat wird abfiltriert, einrotiert und bei 60°C im Hochvakuum andestilliert (ca. 1 h). Der Rückstand besteht aus 3'-Phenoxybenzyl-α-isopropyl-4-difluormethoxy-phenylacetat. $n_D^{20}$ = 1,530.

Beispiel 2

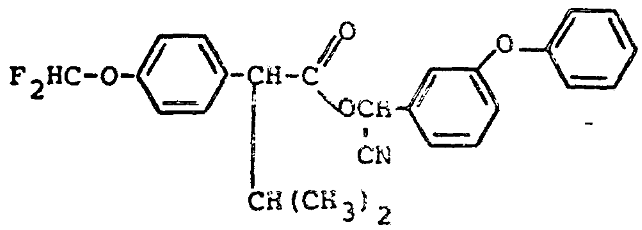

4,1 g 4-Difluormethoxy-α-isopropyl-phenylessigsäurechlorid und 3,6 g 3-Phenoxy-α-cyano-benzylalkohol werden mit 100 ml Toluol vermischt. Dann tropft man bei Raumtemperatur unter Rühren 1,27 g Pyridin in 20 ml Toluol zu und rührt 5 h bei Raumtemperatur nach. Dann wird das Reaktionsgemisch mit 150 ml Wasser versetzt, die organische Phase abgetrennt und mit 100 ml Wasser gewaschen. Die organische Phase wird mit Natriumsulfat getrocknet, abfiltriert, einrotiert und bei 60°C im Hochvakuum andestilliert (ca. 1 h). Der Rückstand besteht aus 3'-Phenoxy-(α'-cyano)-benzyl-α-isopropyl-4-difluormethoxy-phenylacetat. $n_D^{20}$ = 1,540.

Beispiel 3

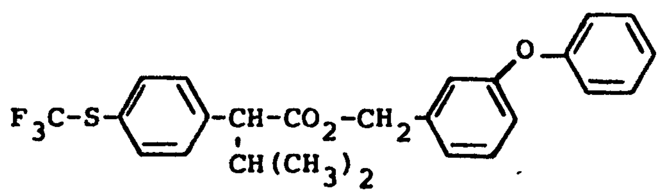

5,9 g 4-Trifluormethylthio-α-isopropyl-phenylessigsäure, 3,7 g 3-Phenoxybenzylchlorid, 100 ml Toluol, 0,5 g Tetrabutylammoniumbromid und 1,08 g pulverisierte KOH (technisch, 88 %ig) werden gemischt und 4 h zum Sieden erhitzt. Nach dem Abkühlen wird die organische Phase abgetrennt und neutral gewaschen. Nach dem Trocknen mit Natriumsulfat wird abfiltriert, einrotiert und bei 60°C im Hochvakuum andestilliert (ca. 1 h). Der Rückstand besteht aus 3'-Phenoxybenzyl-α-isopropyl-4-trifluormethylthiophenylacetat. $n_D^{20}$ = 1,554.

Analog Beispiel 2 werden erhalten:

Beispiel 4

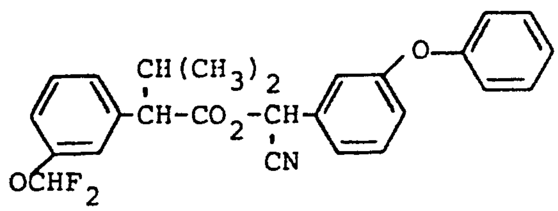

Beispiel 5

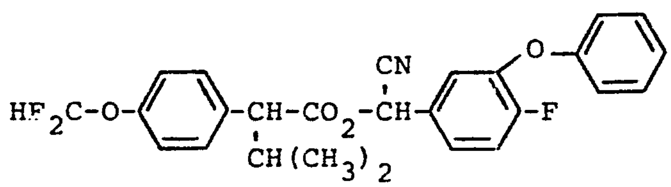

Beispiel 6

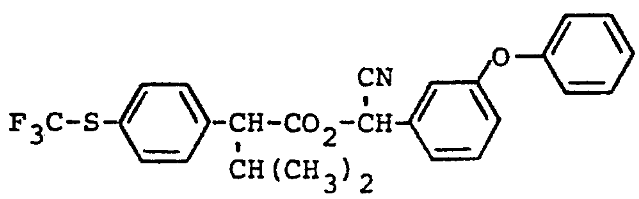

Beispiel 7

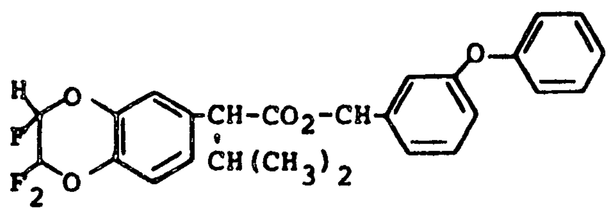

3,4 g 3,4-Trifluoräthylendioxy-α-isopropyl-phenylessigsäurechlorid und 2,2 g 3-Phenoxy-α-cyano-benzylalkohol werden mit 100 ml Toluol vermischt. Dann tropft man bei Raumtemperatur unter Rühren 0,86 g Pyridin in 20 ml Toluol zu und rührt 4 h bei Raumtemperatur nach. Dann wird das Reaktionsgemisch mit 150 ml Wasser versetzt, die organische Phase abgetrennt und mit 100 ml Wasser nachgewaschen. Die organische Phase wird mit Natriumsulfat getrocknet, abfiltriert, einrotiert und bei 60°C im Hochvakuum andestilliert (ca. 1 h). Der Rückstand besteht aus 3'-Phenoxybenzyl-α-isopropyl-3,4-trifluoräthylendioxy-phenylacetat. $n_D^{20}$ = 1,5391.

Beispiel 8

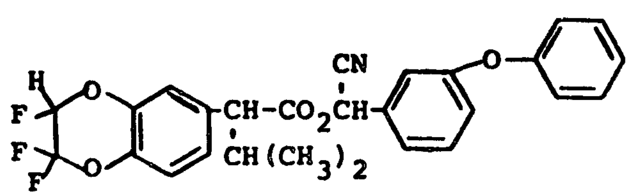

Analog Beispiel 2 wird aus 3,4 g 3,4-Trifluoräthylendioxy-α-isopropyl-phenylessigsäurechlorid und 2,5 g 3-Phenoxy-α-cyano-benzylalkohol und 0,86 g Pyridin 3'-Phenoxy-(α'-cyano)benzyl-α-isopropyl-3,4-trifluoräthylendioxy-phenylacetat erhalten. $n_D^{20}$ = 1,5391.

Analog werden erhalten:

| Beispiel | Formel |
| --- | --- |
| 9 | 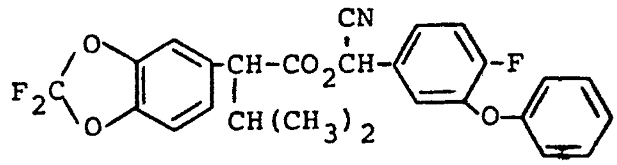 |
| 10 | 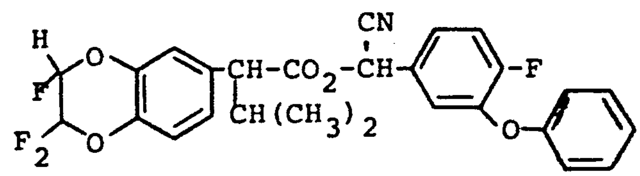 |
| 11 | 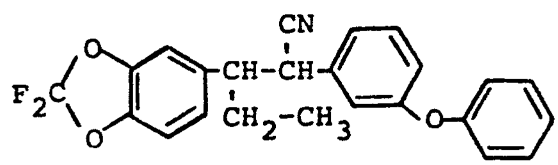 |
| 12 | 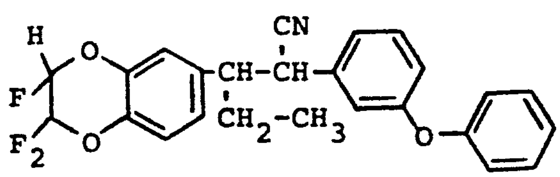 |

**Beispiel 13** 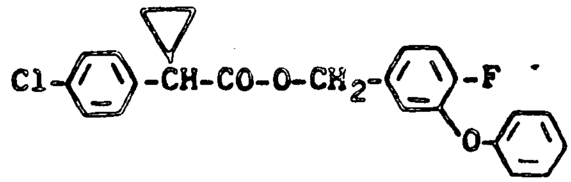

9,4 g (0,043 Mol) 3-Phenoxy-4-fluoro-benzylalkohol und 9,9 g (0,043 Mol) α-Cyclopropyl-4-chlorphenyl-essigsäurechlorid werden in 100 ml wasserfreiem Toluol gelöst und bei 20-25°C 4 g Pyridin, gelöst in 50 ml wasserfreiem Toluol, unter Rühren zugetropft. Anschließend wird weitere 3 Stunden bei 25°C gerührt. Das Reaktionsgemisch wird in 150 ml Wasser, dem 10 ml konzentrierte Salzsäure zugesetzt werden, gegossen, die organische Phase abgetrennt und nochmals mit 100 ml Wasser gewaschen. Anschließend wird die Toluolphase über Natriumsulfat getrocknet und das Lösungsmittel im Wasserstrahlvakuum abdestilliert. Letzte Lösungsmittelreste werden durch kurzes Andestillieren bei 60°C/1 Torr Badtemperatur entfernt. Man erhält 14,9 g (84,4 % der Theorie) α-Cyclopropyl-4-chlorphenyl-essigsäure-3-phenoxy-4-fluoro-benzylester als gelbes Öl. Die Struktur wird durch das H-NMR-Spektrum bestätigt.

**Beispiel 14** 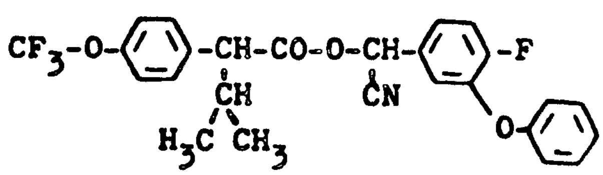

8,64 g (0,04 Mol) 3-Phenoxy-4-fluoro-benzaldehyd und 11,22 g (0,04 Mol) α-Isopropyl-4-trifluormethoxyphenyl-essigsäurechlorid werden zusammen unter Rühren

bei 2o-25°C zu einer Mischung von 3 g Natriumcyanid, 4,7 ml Wasser, 2oo ml n-Hexan und 1 g Tetrabutylammoniumbromid getropft und dann 4 Stunden bei 2o-25°C gerührt. Anschließend wird die Reaktionsmischung mit 3oo ml Toluol versetzt und 2 mal mit je 3oo ml Wasser ausgeschüttelt. Die organische Phase wird abgetrennt, über Magnesiumsulfat getrocknet und das Lösungsmittel im Wasserstrahlvakuum abdestilliert. Letzte Lösungsmittelreste werden durch kurzes Andestillieren bei 6o°C/1 Torr Badtemperatur entfernt. Man erhält 15,6 g (8o,1 % der Theorie) α-Isopropyl-4-trifluormethoxy-phenylessigsäure-3-phenoxy-4-fluoro-α-cyanobenzylester als gelbes Öl mit dem Brechungsindex $n_D^{29}$: 1,5185.

Analog Beispiel 13 oder Beispiel 14 können die folgenden Verbindungen hergestellt werden:

0008732



| Bei-spiel Nr. | Produktformel | Ausbeute (% der Theorie) | Berechungs-index: |
|---|---|---|---|
| 15 | 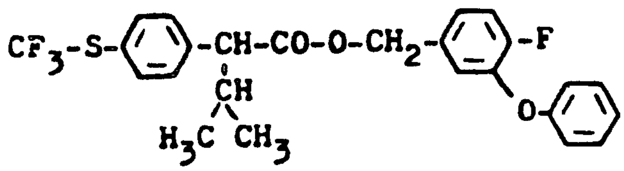 | 70 | $n_D^{27}$: 1,5361 |
| 16 | 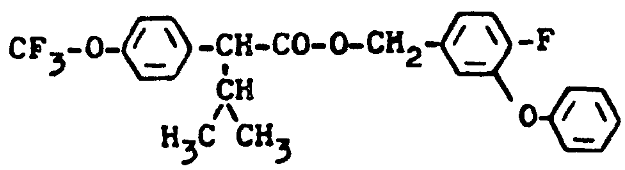 | 83 | $n_D^{27}$: 1,5172 |
| 17 | 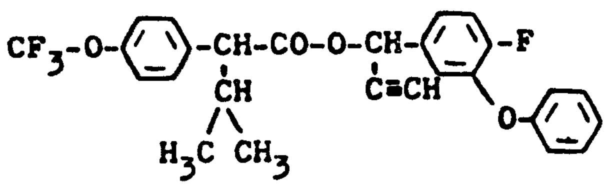 | 72 | $n_D^{27}$: 1,5246 |
| 18 | 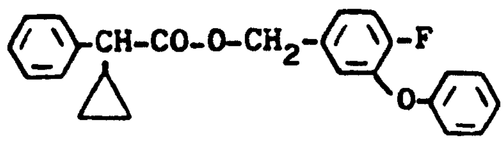 | | |
| 19 | 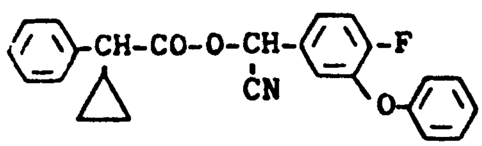 | | |

| Beispiel Nr. | Produktformel | Ausbeute (% der Theorie) | Brechungsindex: |
|---|---|---|---|
| 20 | 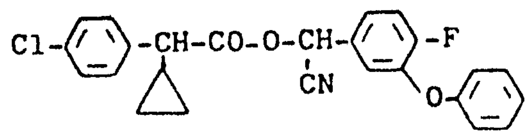 | 79 | |
| 21 | 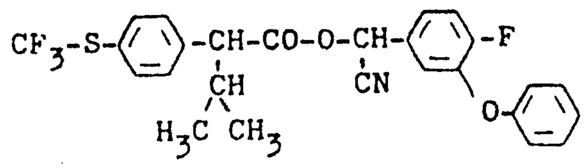 | 72 | $n_D^{27}$:1,5407 |
| 22 | 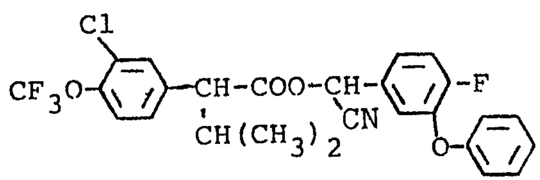 | 76 | |
| 23 | 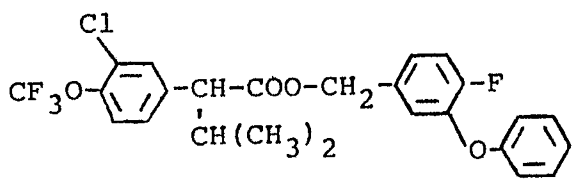 | 78 | |

2. Verbindungen der Formel (II)

Beispiel I

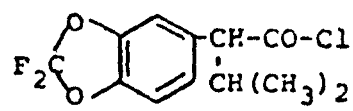

Man löst 30 g 3,4-Difluormethylendioxy-α-isopropyl-phenylessigsäure in 150 ml Thionylchlorid und erhitzt 1 h zum Sieden. Nach Abdestillieren des überschüssigen Thionylchlorids wird der Rückstand im Hochvakuum destilliert. Man erhält das 3,4-Difluormethylendioxy-α-isopropyl-phenylessigsäurechlorid vom Siedepunkt 83 - 86°C/0,3 mm Hg.

Analog werden erhalten:

| Formel | Siedepunkt |
| --- | --- |
| 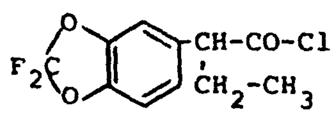 | 74 - 76°C/0,15 mm |
| 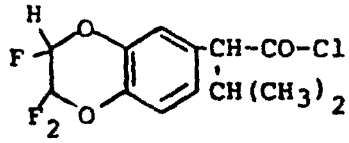 | 88-92°C/0,3 mm |
| 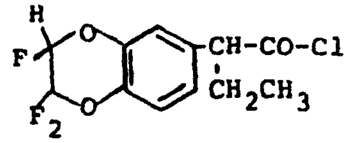 | 80-82°C/0,25 mm |

Beispiel II

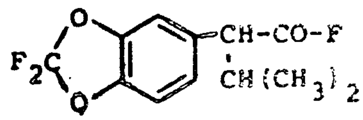

100 ml wasserfreie Flußsäure werden bei -10°C vorgelegt und dann 50 g 3,4-Dichlormethylendioxy-α-isopropyl-phenylessigsäurechlorid zudosiert. Nach Ende der Zugabe wird noch auf 30°C erwärmt und bis zum Ende der Chlorwasserstoffentwicklung gerührt. Dann wird die überschüssige Flußsäure abdestilliert. Der Rückstand besteht aus 3,4-Difluormethylendioxy-α-isopropyl-phenylessigsäurefluorid, das durch Destillation im Hochvakuum gereinigt wird.

Beispiel III

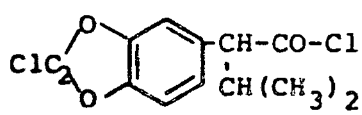

a) 111 g 3,4-Dioxymethylen-α-isopropyl-phenylesigsäure wird mit 350 g $PCl_5$ vermischt. Über eine Kolonne mit Kolonnenkopf wird das beim Erhitzen auf 125°C gebildete $PCl_3$ + $POCl_3$ laufend abdestilliert. Der Rückstand kann direkt wie oben beschrieben weiterverarbeitet werden.

b) 120 g 3,4-Dioxymethylen-α-isopropyl-phenylessigsäurechlorid wird in 200 ml $CCl_4$ gelöst und unter Belichtung bei 80°C 80 g Chlor eingeleitet. Nach Durchleiten von Stickstoff, wird das Lösungsmittel i.V. abgezogen und der Rückstand wie in Beispiel 13 beschrieben, weiterverarbeitet.

Beispiel IV

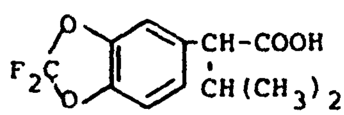

231 g 3,4-Difluormethylendioxy-α-isopropyl-phenylessigsäurenitril werden in 500 ml Äthylenglykol gelöst und mit 80 ml Wasser und 160 g technischer pulverisierter KOH versetzt und 5 h zum Sieden erhitzt. Nach dem Abkühlen wird mit 1500 ml Wasser versetzt und zweimal mit Methylenchlorid extrahiert. Die Wasserphase wird mit konz. HCl unter Kühlung auf pH 1 gestellt und dreimal mit Methylenchlorid extrahiert. Die drei Auszüge werden vereinigt, mit Natriumsulfat getrocknet und einrotiert. Der ölige Rückstand wird,wie weiter oben beschrieben, weiter verarbeitet.

Analog diesem Beispiel werden erhalten:

| Beispiel | Formel |
|---|---|
| | 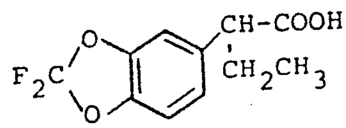 |
| | 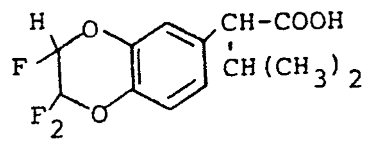 |
| | 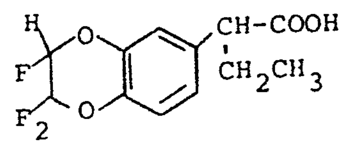 |

Im Falle der letzten beiden Verbindungen wird die saure Wasserphase statt mit Methylenchlorid mit Essigsäureäthylester extrahiert.

Beispiel V

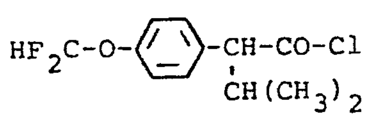

Man löst 20 g 4-Difluormethoxy-α-isopropyl-phenylessigsäure in 100 ml Thionylchlorid und erhitzt 1 h zum Sieden. Nach Abdestillieren des überschüssigen Thionylchlorids wird der Rückstand im Hochvakuum destilliert. Man erhält das 4-Difluormethoxy-α-isopropyl-phenylessigsäurechlorid vom Siedepunkt 93 - 99°C/0,2 mm Hg.

Analog vorstehendem Beispiel wird 3-Difluormethoxy-α-isopropyl-phenylessigsäurechlorid, sowie 4-Trifluormethylthio-α-isopropyl-phenylessigsäurechlorid erhalten.

Beispiel VI

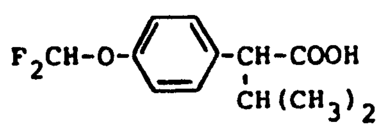

45 g 4-Difluormethylendioxy-α-isopropyl-phenylessigsäurenitril werden in 200 ml trockenem Methanol gelöst und bei $0^{o}$ - $10^{o}C$ mit Chlorwasserstoff gesättigt. Nach Stehen über Nacht wird mit Wasser versetzt und einige Zeit gerührt. Nach Abdestillieren des Methanols wird die Wasserphase mit Methylenchlorid extrahiert. Der Methylester kann durch Destillation gereinigt werden (Siedepunkt: $80^{o}C$/ 0,2 mm) oder aber direkt mit der 4-fachen Menge 15 %iger methanolischer Kalilauge durch 4-stündiges Rückflußkochen verseift werden. Man verdünnt mit Wasser und extrahiert zweimal mit Methylenchlorid. Die Wasserphase wird mit konz. HCl angesäuert und ebenfalls zweimal mit $CH_2Cl_2$ extrahiert. Die beiden letztgenannten Extrakte werden mit Natriumsulfat getrocknet, abfiltriert und einrotiert.

Man erhält die 4-Difluormethylendioxy-α-isopropylphenylessigsäure vom Schmelzpunkt $63^{o}C$.

Analog diesem Beispiel werden erhalten 3-Difluormethoxy-α-isopropyl-phenylessigsäure und 4-Trifluormethylthio-α-isopropylphenylessigsäure.

3. Herstellung von Verbindungen der Formel (IV)

Beispiel VII

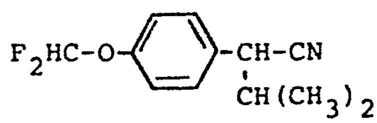

Man legt 98,5 g 4-Difluormethoxyphenylacetonitril, 133 g 2-Brompropan sowie 20 g Tetrabutylammoniumbromid vor und erhitzt zum Sieden. Dann tropft man innerhalb von 2 h 121 g 50 %ige KOH zu und erhitzt anschließend noch 8 h zum Sieden. Nach dem Abkühlen versetzt man mit Wasser, stellt neutral, trennt die organische Phase ab und destilliert. Das 4-Difluormethoxy-α-isopropyl-phenylacetonitril siedet bei 102 - 109°C/0,3 mm Hg.

Das analog erhaltene 3-Difluormethoxy-α-isopropyl-phenylacetonitril siedet bei 98 - 102°C/0,2 mm Hg.

Beispiel VIII

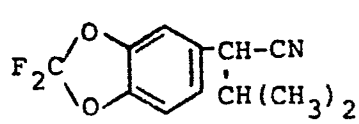

Man legt 78,7 g 3,4-Difluormethylendioxy-phenylacetonitril, 60 g Brompropan sowie 5 g Tetrabutylammoniumbromid vor und erhitzt zum Sieden. Dann tropft man innerhalb von 2 h 54 g 50 %ige Kalilauge zu und erhitzt anschließend noch 8 h zum Sieden. Nach dem Abkühlen versetzt man mit Wasser, stellt neutral, trennt die organische Phase ab und destilliert. Das 3,4-Difluormethylendioxy-α-isopropyl-phenylacetonitril siedet bei 97 - 100°C/0,2 mm Hg.

Beispiel IX

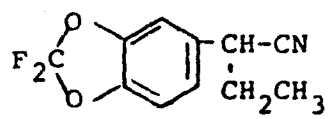

Man arbeitet wir in Beispiel 19, verwendet jedoch statt 2-Brompropan 78 g 2-Jodäthan.

Beispiel X

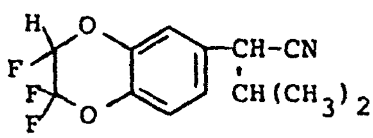

297,5 g 3,4-Trifluoräthylendioxy-phenylacetonitril, 200 g 2-Brompropan und 30 g Tetrabutylammoniumbromid werden zum Sieden erhitzt. Dann tropft man innerhalb von 3 - 4 h 180 g 50 %ige Kalilauge zu. Anschließend

wird noch 6 h weitergekocht. Nach dem Abkühlen versetzt man mit Wasser, stellt neutral, trennt die organische Phase ab und destilliert. Das 3,4-Trifluoräthylendioxy-α-isopropyl-phenylacetonitril siedet bei 105 - 110°C/0,1 mm Hg.

Beispiel XI

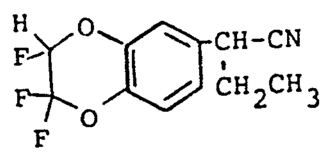

Es wird analog dem vorstehenden Beispiel gearbeitet, jedoch statt 200 g 2-Brompropan werden 300 g 2-Jodäthan verwendet. Kp. = 98 - 102°C/0,15 mm.

Beispiel XII

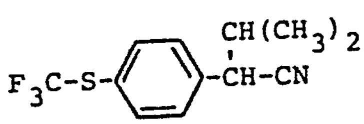

Man legt 235 g 4-Trifluormethylthiophenylacetonitril, 160 g 2-Brompropan, sowie 20 g Tetrabutylammoniumbromid vor und erhitzt zum Sieden. Dann tropft man innerhalb von 3 h 145,5 g 50 %ige KOH zu und erhitzt anschließend noch 3 h zum Sieden. Die Aufarbeitung erfolgt wie im vorstehenden Beispiel. Das 4-Trifluormethylthio-α-isopropyl-phenylacetonitril siedet bei 97 - 103°C/0,3 mm Hg.

4. Herstellung der Verbindungen der Formel (VI)

Beispiel XIII

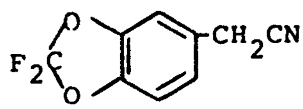

157 g 3,4-Difluormethylendioxy-benzylbromid, 5 g Tetrabutylammoniumbromid und eine 30 %ige wäßrige Natriumcyanidlösung, die 34,3 g NaCN enthält werden 1 h auf 100°C erhitzt. Nach dem Abkühlen extrahiert man zweimal mit Methylenchlorid, trocknet die organische Phase mit Natriumsulfat, filtriert ab und destilliert. Das 3,4-Difluormethylendioxy-phenylacetonitril siedet bei 104 - 108°C/0,4 mm Hg.

Beispiel XIV

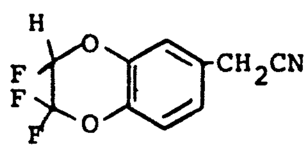

Man löst 135 g Kaliumcyanid in 225 ml Wasser und fügt nacheinander 20 g Tributylamin und 350 g 3,4-Trifluoräthylendioxybenzylchlorid hinzu. Man erhitzt für 1 h unter Rühren auf 100°C, kühlt ab, extrahiert zweimal mit Methylenchlorid, trocknet die organische Phase mit Natriumsulfat, filtriert ab und destilliert. Das 3,4-Trifluoräthylendioxy-phenylacetonitril siedet bei 109 - 110°C/0,01 mm Hg.

Beispiel XV

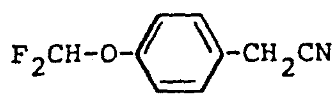

4-Difluormethoxy-benzylcyanid

A) Man legt 270 g 4-Difluormethoxytoluol in 800 ml $CCl_4$ vor und trägt 300 g N-Brom-succinimid ein. Nach Zugabe einer Spatelspitze Azoisobutyronitril wird auf Rückfluß erhitzt. Nach 8 Stunden wird abgekühlt, das unlösliche Succinimid abfiltriert und der Filterrückstand mit $CCl_4$ gewaschen. Aus der Reaktionslösung wird der Tetrachlorkohlenstoff abdestilliert und das rohe Produkt durch fraktionierte Destillation gereinigt. Man erhält 250 g 4-Difluormethoxy-benzylbromid. $Kp_{0,4}$: 82 - 5°C $n_D^{20}$= 1,5200.

B) Man löst 100 g Kaliumcyanid in 160 ml Wasser und fügt anschließend 15 g Tetrabutylammoniumbromid und 230 g 4-Difluormethoxy-benzylbromid hinzu. Dann wird für 1 Stunde auf 100°C erhitzt und nach dem Abkühlen die Mischung zweimal mit Methylenchlorid extrahiert. Die organische Phase wird nach dem Trocknen über Natriumsulfat zuerst vom Lösungsmittel befreit und anschließend destilliert. Man erhält 145 g 4-Difluormethoxy-benzylcyanid. $Kp_{0,3}$: 106-8°C, $n_D^{20}$ = 1,4822.

Die 3-Difluormethoxyverbindungen erhält man analog vorstehendem Beispiel.

Beispiel XVI

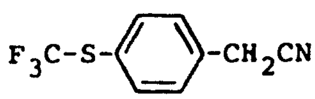

4-Trifluormethylmercapto-benzylcyanid

A) In einer Chlorierungsapparatur aus Glas werden 700 g 4-Trifluormethylmercapto-toluol in 2000 ml $CCl_4$ vorgelegt und auf Rückfluß erhitzt. Die Lösung wird mit einer UV-Lampe bestrahlt. Vor Beginn des Chlorierens werden 3 ml Brom zugesetzt. Nachdem die Farbe des Broms verschwunden ist, werden 200 g Chlor in 6 Stunden eingeleitet. Durch fraktionierte Destillation über eine 40 cm Füllkörper-Kolonne werden neben nicht chloriertem Ausgangsmaterial 560 g 4-Trifluormethylmercaptobenzylchlorid erhalten. Kp: 97-8°C/ 14 mm, $n_D^{20}$ = 1,5072.

B) Man löst 75 g Natriumcyanid in 200 ml Wasser und fügt 15 g Tetraäthylammoniumchlorid nach 450 g 4-Trifluormethylmercapto-benzylchlorid hinzu. Die Reaktionsmischung wird für 1 Stunde bei 110°C gerührt und anschließend abgekühlt. Der kalte Ansatz wird mit 200 ml Wasser verdünnt und dann zweimal mit Methylenchlorid extrahiert. Nach dem Trocknen der organischen Phase wird das Lösungsmittel abdestilliert und das Rohprodukt durch Destillation gereinigt. Man erhält 410 g 4-Trifluormethylmercapto-benzylcyanid. Kp: 95 - 97°C/1,5 mm, $n_D^{20}$ = 1.5025.

5. Herstellung der Verbindungen der Formel (VIII)

Beispiel XVII

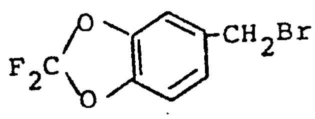

172 g 3,4-Difluormethylendioxytoluol, 180 g N-Bromsuccinimid und eine Spatelspitze Azobisisobutyronitril werden mit 1000 ml $CCl_4$ gemischt und 5 h zum Sieden erhitzt. Nach dem Abkühlen wird abfiltriert, mit etwas $CCl_4$ nachgewaschen und das Filtrat destilliert. Man erhält 3,4-Difluormethylendioxy-benzylbromid vom Kp = 108 - 111°C/15 mm.

Beispiel XVIII

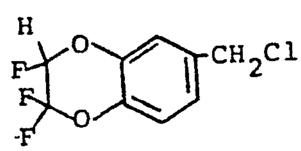

In einer Chlorierungsapparatur aus Glas werden 1000 g 3,4-Trifluoräthylendioxy-toluol bei 120°C vorgelegt und mit einer UV-Lampe bestrahlt. Dann werden innerhalb von 7 h 300 g Chlor eingleitet. Nach Ende des Einleitens wird noch für 20 Minuten nachreagieren lassen und anschließend kurz mit Stickstoff ausgeblasen.

Durch Destillation über eine Füllkörperkolonne werden erhalten:

195 g Ausgangsmaterial Kp = 76°/15 mm
58 g Zwischenlauf Kp = 77-115°/15 mm
645 g 3,4-Trifluoräthylendioxy-benzylchlorid vom Kp = 115°C/15 mm ($n_D^{20}$ = 1,4906)

6. Herstellung der Ausgangsverbindungen bzw. Herstellung der Verbindungen der Formel (VIII)

Beispiel XIX

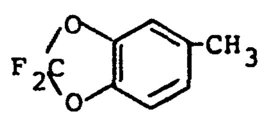

Im Reaktionsgefäß werden bei 0°C 200 ml HF (wasserfrei) vorgelegt und 190 g 3,4-Dichlormethylendioxytoluol (vgl. J. Chem. Soc. 93, 563) zugetropft. Nach Ende der Chlorwasserstoffentwicklung wird auf 20°C erwärmt, 1 h nachgerührt und anschließend die überschüssige HF bei vermindertem Druck abdestilliert. 3,4-Difluormethylendioxy-toluol siedet bei 74-78°C/52 mm Hg.

Beispiel XX

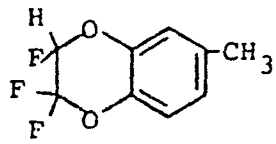

124 g 4-Methylbrenzkatechin werden zusammen mit 110 g Kaliumhydroxid in 300 ml Tetramethylensulfon bei 110°C vorgelegt. Es werden dann innerhalb von 4 h 170 g Trifluorchloräthylen eingeleitet. Der Ansatz wird anschließend bei 15 mm über eine Kolonne destilliert, wobei bis zu einer Übergangstemperatur von 85°C abgenommen wurde. Nach Abtrennen der wäßrigen Phase in der Vorlage wird das Produkt erneut destilliert. Man erhält bei einem Siedepunkt $Kp_{12}$ = 70 - 72°C 3,4-Trifluoräthylendioxy-toluol ($n_D^{20}$ = 1,4565).

Patentansprüche

1. Fluorhalige Phenylessigsäureester der Formel (I)

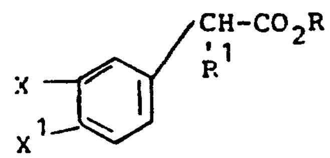

(I)

in welcher

R für den Rest eines bei Pyrethroiden üblichen Alkohols steht,

$R^1$ für $C_{2-4}$-Alkyl, $C_{2-4}$-Alkenyl oder Cyclopropyl steht,

X für H, Halogen, Alkyl, Alkoxy, $OCHF_2$, $SCHF_2$, $SCClF_2$, $SCF_3$ steht,

$X^1$ für den Fall, daß R für gegebenenfalls durch α-Cyano, oder α-Äthinylsubstituierten 4-Fluor-3-phenoxybenzylalkohol steht, für Halogenalkoxy oder Halogenalkylthio oder gemeinsam mit X für fluorsubstituiertes Methylendioxy oder Ethylendioxy steht und zusätzlich für den Fall, daß $R^1$ für Cyclopropyl steht und R für gegebenenfalls durch α-Cyano oder α-Äthinyl substituierten 4-Fluor-3-phenoxybenzylalkohol steht, für Wasserstoff, Halogen, Alkyl, Halogenalkyl, Alkoxy, Alkylthio oder gemeinsam mit x für gegebenenfalls F substituiertes Methylendioxy oder für gegebenenfalls F substituiertes Ethylendioxy steht und in den Fällen, in denen R für andere bei Pyrethroiden übliche Alkohole steht als für gegebenenfalls durch α-Cyano oder α-Äthinylsubstituierten 4-Fluor-3-phenoxybenzylalkohol, für Wasserstoff, $OCHF_2$, $SCHF_2$, $SCClF_2$, $SCF_3$, sowie ge-

gemeinsam mit X für $-OCH_2CF_2O-$ oder $-OCHFCF_2O-$ steht wobei in diesen Fällen X für $OCHF_2$, $SCHF_2$, $SCClF_2$ oder $SCF_3$ stehen muß wenn $X^1$ für Wasserstoff steht.

2. Verfahren zur Herstellung der fluorhaltigen Phenylessigsäureester der Formel (I), dadurch gekennzeichnet, daß man eine Säure oder deren reaktionsfähiges Derivat der Formel (II)

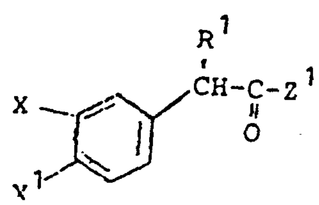

(II)

in welcher

X und $R^1$ die unter 1. (oben) angegebene Bedeutung haben und

$Z^1$ Halogen, vorzugsweise Fluor oder Chlor, OH oder $OC_{1-4}$-Alkyl bedeutet,

mit einem Äthanol oder dessen reaktionsfähigen Derivat der Formel (III)

$$R\text{-}Z^2 \quad \text{(III)}$$

in welcher

R die unter 1. (oben) angegebene Bedeutung hat und

$Z^2$ OH, Cl oder Br bedeutet,

gegebenenfalls in Gegenwart von Lösungsmitteln, Säureakzeptoren und/oder Phasentransferkatalysatoren

oder in Gegenwart von Umesterungskatalysatoren umsetzt.

3. Säuren bzw. die reaktionsfähigen Derivate derselben der Formel (XII)

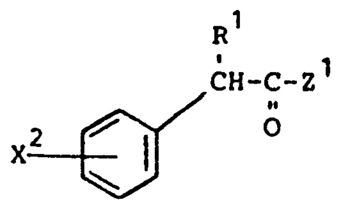

(XII)

in welcher

$X^2$ in 3 und/oder 4 Stellung für die Reste $-OCHF_2$, $-SCHF_2$, $-SCClF_2$, $-SCF_3$ sowie in 3 und 4 Stellung für die Reste $-OCH_2CF_2O-$ oder $-OCHFCF_2O-$, steht,

$R^1$ für $C_2$-$C_4$-Alkyl $C_{2-4}$-Alkenyl oder Cyclopropyl steht und

$Z^1$ die unter 2. (oben) angegebene Bedeutung besitzen.

4. Verfahren zur Herstellung der Säuren, bzw. der reaktionsfähigen Derivate derselben, der Formel (XII), dadurch gekennzeichnet, daß man die Verbindungen der Formel (IV)

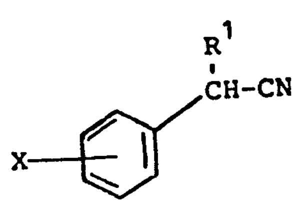

(IV)

in welcher

X und $R^1$ die unter 1. (oben) angegebene Bedeutung besitzen,

mit einem Alkohol der Formel (V)

$$C_{1-4}\text{-Alkyl-OH} \qquad \text{(V)}$$

in Gegenwart eines sauren Katalysators umsetzt und den entstandenen Iminoester zum Ester hydrolysiert, diesen gegebenenfalls zur Säure verseift und diese gegebenenfalls mit einem Halogenierungsmittel umsetzt oder für den Fall, daß X in 3 und 4 Stellung für die Reste $-OCH_2CF_2O-$, $OCHFCF_2O-$ steht die CN-Gruppe der Verbindung der Formel (IV) zur COOH-Gruppe verseift und diese gegebenenfalls mit einem Halogenierungsmittel umsetzt.

5. Verbindungen der Formel (IV)

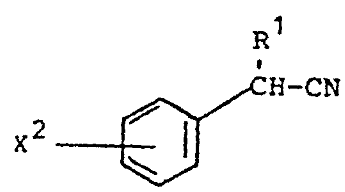

(IV)

in welcher

$X^2$ die unter 3. (oben) angegebene Bedeutung besitzen.

6. Verfahren zur Herstellung der Verbindungen der Formel (IV), dadurch gekennzeichnet, daß man die Verbindungen der Formel (VI)

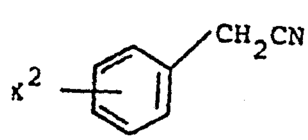

(VI)

in welcher

X die unter 3. (oben) angegebene Bedeutung besitzt,

mit einer Verbindung der allgemeinen Formel (VII)

$R^1$-Hal (VII)

in welcher

$R^1$ die unter 1. (oben) angegebene Bedeutung besitzt und

Hal für Cl, Bro oder J steht,

in Gegenwart einer Base, sowie gegebenenfalls in Gegenwart eines Lösungsmittel und/oder eines Phasentransferkatalysators umsetzt.

7. Verbindungen der Formel (VI)

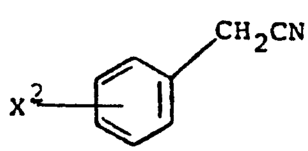

(VI)

in welcher

X die unter 3. (oben) angegebene Bedeutung besitzt.

8. Verfahren zur Herstellung der Verbindungen der Formel (VI), dadurch gekennzeichnet, daß man die Verbindungen der Formel (VIII)

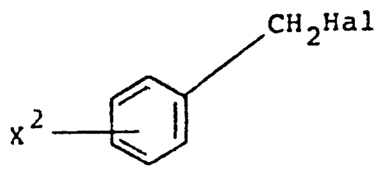

(VIII)

in welcher

$X^2$ die unter 3. (oben) angegebene Bedeutung besitzt und

Hal für Cl oder Br steht,

mit Cyanwasserstoff oder vorzugsweise einem Alkalicyanid, gegebenenfalls in Gegenwart eines Katalysators umsetzt.

9. Verfahren zur Herstellung der Säurederivate der Formel (II) in welcher

X und $X^1$ in 3 und 4-Stellung für den Rest $-OCF_2O-$ stehen.

$Z^1$ für Fluor steht und

$R^1$ für $C_2$-$C_4$-Alkyl steht,

dadurch gekennzeichnet, daß man eine Verbindung der Formel (IX)

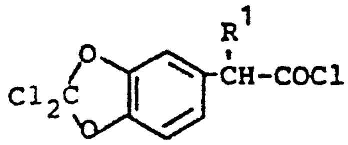

(IX)

in welcher

$R^1$ für $C_{2-4}$-Alkyl steht,

mit wasserfreier Flußsäure umsetzt.

10. Verbindungen der Formel (IX)

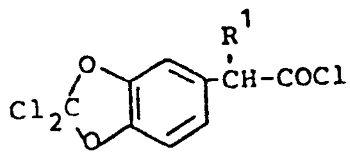

(IX)

in welcher

$R^1$ für $C_{2-4}$-Alkyl steht.

11. Verfahren zur Herstellung der Verbindungen der Formel (IX), dadurch gekennzeichnet, daß man eine Verbindung der Formel (X)

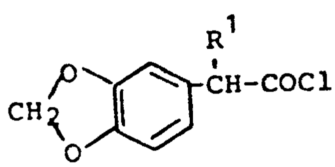

(X)

in welcher

$R^1$ für $C_{2-4}$-Alkyl steht,

mit einem Chlorierungsmittel umsetzt.

12. Insektizide und/oder akarizide Mittel, gekennzeichnet durch einen Gehalt an mindestens einem fluorhaltigen Phenylessigsäureester der Formel (I).

13. Verwendung von fluorhaltigen Phenylessigsäureester der Formel (I) zur Bekämpfung von Insekten und/oder Spinnentieren.